(19) Europäisches Patentamt
European Patent Office
Office européen des brevets

(11) Veröffentlichungsnummer : **0 442 372 B1**

(12) **EUROPÄISCHE PATENTSCHRIFT**

(45) Veröffentlichungstag der Patentschrift :
**03.05.95 Patentblatt 95/18**

(51) Int. Cl.[6] : **G01N 33/532,** G01N 33/533, G01N 33/534, G01N 33/535, G01N 33/78

(21) Anmeldenummer : **91101656.6**

(22) Anmeldetag : **07.02.91**

(54) **Verbesserte markierte Haptene, Verfahren zu deren Herstellung sowie Verwendung dieser markierten Haptene in Immunoassays.**

(30) Priorität : **13.02.90 DE 4004296**

(43) Veröffentlichungstag der Anmeldung :
**21.08.91 Patentblatt 91/34**

(45) Bekanntmachung des Hinweises auf die Patenterteilung :
**03.05.95 Patentblatt 95/18**

(84) Benannte Vertragsstaaten :
**AT BE CH DE DK ES FR GB IT LI NL**

(56) Entgegenhaltungen :
**EP-A- 0 135 071**
**WO-A-86/04145**
**DE-A- 3 628 795**
**US-A- 4 810 638**
**CHEMICAL ABSTRACTS, Band 111, Nr. 12, 18. September 1989, Columbus, OH (US); T. ITOH et al., Seite 351, Nr. 102661u**

(73) Patentinhaber : **HOECHST AKTIENGESELLSCHAFT**
**D-65926 Frankfurt (DE)**

(72) Erfinder : **Kinkel, Tonio, Dr.**
**Feldbergstrasse 14**
**W-6239 Kriftel (DE)**
Erfinder : **Mayer, Andreas, Dr.**
**Rosenpark 32**
**W-6234 Hattersheim am Main (DE)**
Erfinder : **Neuenhofer, Stephan, Dr.**
**Falkensteiner Weg 16**
**W-6231 Sulzbach (DE)**
Erfinder : **Oekonomopulos, Raymond, Dr.**
**Lessingweg 83**
**W-6200 Wiesbaden (DE)**

EP 0 442 372 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Immunoassays für Haptene bestehen im allgemeinen aus einem für das jeweilige Hapten spezifischen Antikörper und einem markierten Hapten-Konjugat. Dieses Hapten-Konjugat enthält im allgemeinen eine Gruppe (Label), welche durch physikalische oder chemische Methoden nachgewiesen werden kann und gegebenenfalls eine Verknüpfungsgruppe zwischen Hapten und Label. Die Verknüpfungsgruppe kann im einfachsten Fall ersetzt werden durch eine direkte chemische Bindung, jedoch ist es üblich, durch eine Verknüpfungsgruppe einen gewissen räumlichen Abstand zu erzielen. Es hat sich gezeigt, daß bei einer direkten Kopplung des Haptens mit einer nachweisbaren Gruppe (Label) die Eigenschaften beider Gruppen so verändert wurden, daß die Empfindlichkeit und Spezifität der Teste verringert wurden. Zum einen veränderten sich die physikalischen Eigenschaften der nachweisbaren Gruppe (Label) und zum anderen die Spezifität und Affinität des Haptens zu seinem Antikörper.

Typische chemilumineszent-markierte Konjugate und diese enthaltende Lumineszenz-Immunoassays sind beispielsweise in der DE-A 29 21 781 beschrieben. Diese Lumineszenz-Immunoassays weisen gegenüber Radioimmunoassays den Vorteil auf, daß man nicht mit radioaktiven Substanzen arbeiten muß, die nur eine begrenzte Lebensdauer haben und wegen der entsprechenden Schutzvorschriften nur begrenzt eingesetzt werden können.

Die DE-A 29 13 549 beschreibt chemisch induzierte Fluoreszenz-Immunoassays, bei welchen der Antiligand spezifisch am epitopen Zentrum des Liganden gebunden wird und als Marker ein lichtemittierendes reziprokes Paar vorgesehen ist, welches aus einer Chemilumineszenzquelle und einem Quencher besteht, der das von der Chemilumineszenzquelle emittierte Licht kollisionsfrei auslöschen kann. Dabei werden Konjugate mit Chemilumineszenzmarkierung und Konjugate mit Quenchermarkierung gebildet und an Bestandteile des immunologischen Paares gebunden. In der Beschreibung wird erwähnt, daß es prinzipiell möglich sei, auch eine Mehrzahl von Markern an dem Bestandteil des immunologischen Paares zu binden, wobei sogenannte polyligandenanaloge Marker entstehen. Besondere Vorteile derartiger Systeme sind jedoch nicht erwähnt. Ein Nachteil besteht darin, daß das analoge Paar den Quencher in vergleichbaren Abständen enthalten muß, um entsprechend wirksam zu sein. Bei den relativ kleinen Haptenen ist die Besonderheit zu beachten, daß diese oftmals zu einer wesentlich verringerten Chemilumineszenz führen, ohne daß zusätzlich ein Quencher an den Rezeptor gebunden ist. Diese Wechselwirkung zwischen Hapten und chemilumineszierender Gruppe ist bereits mehrfach beobachtet worden und führt zu verringerter Empfindlichkeit und Spezifität der Teste, weshalb man in solchen Fällen vorzugsweise mit einem "Spacer" (Abstandshalter) arbeitet.

In der EP-A 0 135 071 werden chemilumineszent markierte Hapten-Konjugate beschrieben, welche zwischen der zur Chemilumineszenz befähigten Gruppe und dem Hapten eine Verknüpfungsgruppe ("Spacer") aufweisen, die ein kettenförmiges Polymeres mit wiederkehrenden funktionellen Gruppen ist, an welches pro Mol Polymer sowohl mehrere Mole zur Lumineszenz befähigte Gruppen, als auch mehrere Mole Hapten gebunden sind. Die dort beschriebenen polymeren Spacer müssen eine ausreichende Anzahl wiederkehrender funktioneller reaktiver Gruppen aufweisen, wie Amino-, Carboxyl-, Carbonyl-, Thionyl-, Hydroxyl- und/oder zur Diazo-Kopplung befähigte Gruppen, über die sowohl die zur Chemilumineszenz befähigten Gruppen als auch die Haptene gebunden werden können.

In der DE-A 36 28 795 (HOE 86/F 209) werden Thyroninderivate beschrieben, an welche jeweils eine mit chemischen oder physikalischen Methoden quantifizierbare Gruppe über einen Polyethylenglykol-Spacer (PEG-Spacer) gebunden ist. Die dort beschriebenen PEG-Spacer weisen mindestens 20 Monomer-Einheiten ($CH_2$-$CH_2$-O) auf, vorgeschlagen werden solche mit 10 - 400 Monomer-Einheiten.

Alle bisher beschriebenen markierten Haptene weisen aber immer noch den Nachteil auf, daß, wahrscheinlich aufgrund der angekoppelten nachweisbaren Gruppen, die Immunreaktivität der Haptene verringert wird und/oder daß die chemische oder physikalische Eigenschaft der nachweisbaren Gruppe durch Wechselwirkung mit dem Hapten oder dem Spacer nachteilig beeinflußt wird. Befriedigende Immunoassays, insbesondere Chemilumineszenz-Immunoassays, konnten bisher mit diesen markierten Haptenen nicht hergestellt werden, da meist die erhaltenen Standardkurven im physiologisch interessierenden Bereich zu flach verliefen.

Aufgabe der vorliegenden Erfindung war es dementsprechend, markierte, insbesondere chemilumineszent markierte Haptene zur Verfügung zu stellen, bei welchen die zuvor erwähnten Nachteile nicht auftreten und bei deren Einsatz in Immunoassays eine hohe Empfindlichkeit und eine gute Reproduzierbarkeit gewährleistet ist.

Diese Aufgabe wurde überraschenderweise gelöst durch markierte Haptene der Formel I

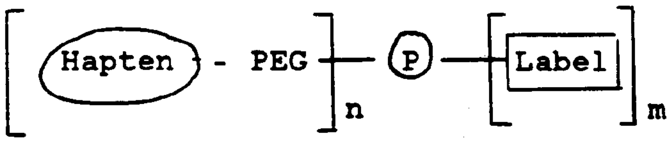 (I)

worin

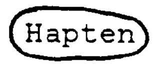

eine biologisch aktive Substanz,

PEG ein Polyethylenglykol der Formel II

$$-\overset{O}{\overset{\|}{C}}-A-(O-CH_2-CH_2)_x-NH- \qquad (II)$$

worin

A $C_1$-$C_3$-Alkylen oder $-CH_2-NHC(O)-$ ist und

x 1 bis 60 ist, oder

PEG ein Rest der Formel III ist

$$\left[\overset{O}{\overset{\|}{C}}-A'-\overset{O}{\overset{\|}{C}}-NH-(CH_2)_o-O-(CH_2)_p-O-(CH_2)_o-NH\right]_y \qquad (III)$$

worin

A' $C_1$-$C_3$-Alkylen oder $-CH_2-O-CH_2-$ ist und

o 2 - 4 und p 1 - 5 ist und

y 1 - 7 ist;

Ⓟ ein Protein, Oligo- oder Polypeptid ist,

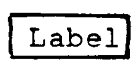

eine mit chemischen oder physikalischen Methoden quantifizierbare Gruppe ist;

n 1 - 4 und

m 1 - 4 ist.

Insbesondere betrifft die Erfindung solche markierten Haptene der Formel I, in denen

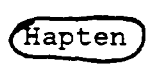

ein Rest der Formel

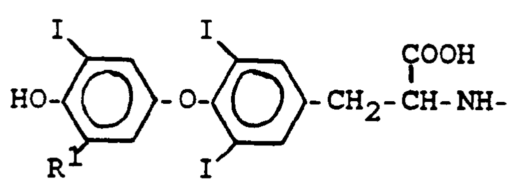

ist

worin

$R^1$ H, I oder ein radioaktives Iodisotop ist und

PEG ein Polyethylenglykol der Formel II ist,
worin
A $-CH_2-$ und
x 3 - 60 ist
oder

PEG ein Rest der Formel III ist
worin
o 3 und
p 4 ist,
y 1 oder 2 ist und
A' $CH_2-CH_2$ oder $CH_2-O-CH_2$ ist

Ⓟ ein Oligo- oder Polypeptid, HSA (Humanserumalbumin), BSA (Rinderserumalbumin) IgG oder ein IgG-Fragment ist

Label

ein chemilumineszentes Acridinium-9-(N-sulfonyl)-carbonsäureamid-Derivat ist und

n 1 - 4 und

m 1 - 4 ist.

Unter "biologisch aktiven Substanzen" oder Haptenen sind vor allem Antigene zu verstehen. Unter diesen Begriff fallen z.B. Hormone, Steroide, Arzneimittel, Arzneimittelmetabolite, Toxine, Alkaloide und auch Antikörper. Bevorzugt handelt es sich hierbei um Triiodthyronin (T3) und Thyroxin (T4).

Bevorzugt eingesetzte Proteine, Oligo- und Polypeptide sind beispielsweise Immunglobuline wie IgG, Serumalbumine wie HSA, BSA, natürliche und synthetische Oligo- und Polypeptide wie Polylysin, Poly (Glu, Lys), Poly (Glu, Lys, Tyr) mit wechselnden Aminosäuregehalten.

Unter einem Label werden chemisch oder physikalisch quantifizierbare Gruppen verstanden wie Radioisotope, radioaktiv markierte Verbindungen, chemilumineszierende oder fluoreszierende Verbindungen, Enzyme oder Substrate; insbesondere chemilumineszierende Acridinium-Derivate wie substituierte Acridinium-9-(N-sulfonyl)-carbonsäureamide, wie sie beispielsweise in der EP 0 330 050 oder EP 0 257 541 (HOE 88/F 042 + HOE 86/F 204) beschrieben sind.

Weiterhin betrifft die Erfindung ein Verfahren zur Herstellung der markierten Haptene der Formel I, wobei man die biologisch aktive Substanz - das Hapten - umsetzt mit einer Verbindung der Formel IV oder V

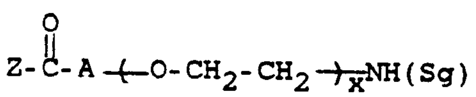 (IV)

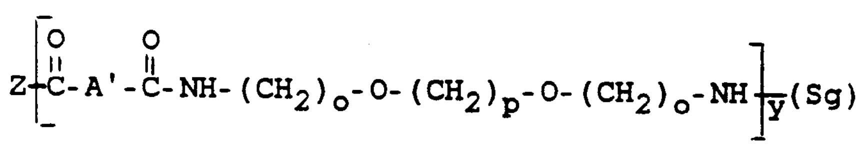 (V)

worin

Z beispielsweise -OH, -Cl,

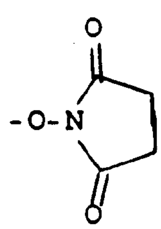 , 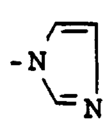 , 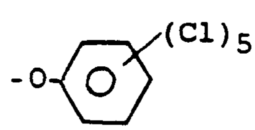 ,

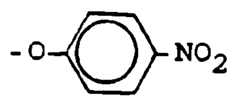 , 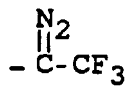 oder 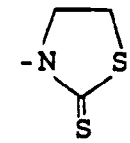

bedeutet und
wobei x, y, A, A′, o und p wie für Formeln II und/oder III definiert sind und (Sg) H oder eine Aminoschutzgruppe ist, und anschließend das so erhaltene Produkt (Hapten-PEG-derivat)

a) gegebenenfalls nach Entfernen der Aminoschutzgruppe zunächst mit einem Protein und dieses Produkt anschließend mit einem Label umsetzt oder

b) gegebenenfalls nach Entfernen der Aminoschutzgruppe mit einem Protein-Label-Konjugat umsetzt

und gegebenenfalls anschließend das Reaktionsprodukt reinigt.

Das als Ausgangssubstanz zur Herstellung von Verbindungen der Formel IV benötigte Polyethylenglykol mit geeigneter Kettenlänge ist käuflich erhältlich. Beispielsweise eignen sich die Typen Polyethylenglykol 200, 300, 400 oder 600. Bei diesen Zahlenangaben handelt es sich um das mittlere Molekulargewicht, z.B. PEG 600: mittlere molare Masse 570-630 g/mol; Hydroxylzahl 178-197 (rechnerisch 187). Aus dem mittlerem Molekulargewicht des Polyethylenglykols läßt sich der Wert x folgendermaßen rechnerisch ermitteln: mittleres Molekulargewicht des Polyethylenglykols (z.B. 600) minus Molekulargewicht $H_2O$ (18), dividiert durch das Molekulargewicht der Monomereinheit ($CH_2$-$CH_2$-O:MG=44); beispielsweise für PEG 600: (600-18)/44=13.2. Zwischenwerte wie PEG 500 erhält man durch Mischen von z.B. einem Teil PEG 400 und einem Teil PEG 600, allerdings erhält man hierbei eine deutliche Verbreiterung der Molmassenverteilung. Eine andere Möglichkeit besteht in der Änderung der Reaktionsführung bei der Polymerisation, um die gewünschte Verteilung zu erhalten. Unter PEG 150 wird Triethylenglykol $H(OCH_2CH_2)_3OH$ verstanden, das in dieser Form oder als Monochlorhydrin $H(OCH_2CH_2)_3$-Cl rein käuflich ist.

Die Herstellung der Verbindung der Formel IV erfolgt in Anlehnung an das oder nach dem von Zalipsky und Barany in Polym. Prep. 27, 1 (1986) beschriebenen Verfahren zur Darstellung von Polyethylenglykolderivaten mit zwei verschiedenen funktionellen Gruppen an den Enden. Dazu wird das eingesetzte Polyethylenglykol zunächst partiell chloriert, beispielsweise mit Thionylchlorid in einem geeigneten Lösungsmittel wie Benzol oder vorzugsweise Toluol und unter Zusatz von säurebindenden Mitteln wie Pyridin. Die Reaktion wird bei 50-150 °C, vorzugsweise bei 110 °C durchgeführt. Das Produkt kann isoliert und gereinigt werden, bevorzugt wird es jedoch ohne Reinigung weiter eingesetzt. Die in der obengenannten Vorschrift beschriebenen Fällungen lassen sich nur bei PEG-Derivaten mit einem Molekulargewicht >1000 anwenden, bei kürzeren PEG's sind die Produkte vorwiegend Öle. Soll eine Urethangruppe (Formel II; A = $-CH_2$-NHC(O)-) eingeführt werden, so wird wie in der oben angegebenen Arbeit (Zalipsky et al., loc. cit.) das Rohprodukt mit Isocyanatoessigsäureestern, bevorzugt Isocyanatoessigsäureethylester in Gegenwart von Triethylamin vorzugsweise bei Raumtemperatur mehrere Stunden zu einer Verbindung der Formel VI umgesetzt.

$$(Cl)_s(CH_2-CH_2-O)_x(A-\overset{\overset{\displaystyle O}{\|}}{C}R^2)_z \qquad (VI)$$

mit

s,z = 0, 1, 2 wobei s + z = 2,
x wie in Formel II definiert,
A = $-C(O)-NH-CH_2-$,
$R_2$ = $C_1$-$C_4$-Alkoxy.

Soll eine Ethergruppierung eingeführt werden, so wird in Abweichung von der obengenannten Verfahrensvorschrift das partiell chlorierte Polyethylenglykol mit einer Verbindung der Formel VII AG-ACOR$^2$ (AG = Abgangsgruppe, wie Cl, Br, I oder $N_2$, A= =CH- im Fall von $N_2$, sonst wie in Formel II definert, $R^2$ = $C_1$-$C_4$-Alkoxy, Li, Na, K) bei Temperaturen von -50 bis +50 °C in einem geeigneten Lösungsmittel wie Diethylether, 1,2-Dimethoxyethan oder tert.-Butanol, gegebenenfalls unter Zusatz einer Base wie n-Butyllithium in Hexan, Natriumhydrid, Kalium-tert.-butylat oder feingepulvertem KOH unter Zugabe eines Phasentransfer-Katalysators wie Tetraalkylammoniumbromid oder eines Katalysators wie $BF_3 \cdot Et_2O$ zu einer Verbindung der Formel VI umgesetzt (x wie in Formel II definiert, A = Alkylen, $CH_2$-O-$CH_2$, $R^2$ wie in Formel VII definiert). Die in den Produkten der Formel VI verbliebenen Chloratome werden anschließend, beispielsweise durch Umsetzung mit $NaN_3$ in einem geeigneten Lösungsmittel wie DMF bei Temperaturen von 50-150 °C, vorzugsweise bei 120

°C, durch eine Azidgruppe ersetzt. Durch Umsetzung mit einer Base, vorzugsweise wäßriger NaOH und anschließendem Ansäuern wird ein Rohprodukt der Formel VIII erhalten (Formel VIII: $(N_3)_s(CH_2\text{-}CH_2O)_x(A\text{-}CO_2H)_z$ , mit x und A wie in Formel II und s, z wie in Formel VI definiert). Bei diesem Produkt handelt es sich um eine Mischung aus PEG-Diazid, PEG-Azid-Carbonsäure und PEG-Dicarbonsäure jeweils mit einer Homologenverteilung. Mittels Ionenaustauscherchromatographie über DEAE-Sephadex (Pharmacia) in der Tetraboratform mit einer stufenweise konzentrierter werdenden wäßrigen Ammoniumhydrogencarbonatlösung kann man die ungewünschten Produkte PEG-Diazid (Formel VIII, s = 2, z = 0) und PEG-Dicarbonsäure (Formel VIII, s = 0, z = 2) von den gewünschten PEG-Azidcarbonsäuren (Formel VIII, s = 1, z = 1) abtrennen. Dieses Produkt wird dann der literaturbekannten katalytischen Hydrierung, beispielsweise mittels $H_2$/Pd/kohle unterworfen, wobei die Azidgruppe in eine Aminogruppe umgewandelt wird, die anschließend zweckmäßigerweise nach literaturbekannten Verfahren geschützt wird, wobei eine Verbindung der Formel IV entsteht. Als Schutzgruppen können die von der Peptidsynthese her bekannten Aminoschutzgruppen verwendet werden, vorzugsweise tBOC (tert.-Butyloxycarbonyl). Der vorstehend beschriebene Syntheseweg ist im Syntheseschema 1 dargestellt.

Zur Herstellung der Verbindungen der Formel V geht man zweckmäßigerweise von einer Verbindung der Formel IX: $H_2N\text{-}(CH_2)_o\text{-}O\text{-}(CH_2)_p\text{-}O\text{-}(CH_2)_o\text{-}NH_2$ (worin o und p wie für Formel III beschrieben sind) aus. Verbindungen der Formel IX, worin o = 3 und p = 4 ist sowie solche, worin o = p = 2 ist, sind käuflich.

Die Verbindung der Formel IX wird in einem geeigneten Lösungsmittel wie Acetonitril bei Temperaturen von 0 °C bis Raumtemperatur mit einer Verbindung der Formel X

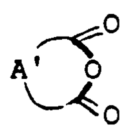

(A′ = siehe Definition in Formel III) zu einer Verbindung der Formel XI:

$$H_2N\text{-}(CH_2)_o\text{-}O\text{-}(CH_2)_p\text{-}O\text{-}(CH_2)_o\text{-}NH\text{-}C(O)\text{-}A'\text{-}COOH$$

umgesetzt. Zweckmäßigerweise wird man auch hier die Aminogruppe mit einer der oben für Verbindungen der Formel IV beschriebenen Aminoschutzgruppen nach literaturbekannten Verfahren schützen, wobei Verbindungen der Formel V entstehen.

Bevorzugt wird nun die Verbindung der Formel IV oder V zunächst an die biologisch aktive Substanz gebunden. Dazu wird die Verbindung der Formel IV (Z = OH) oder V zunächst in eine aktivierte Form überführt, z.B. durch Synthese des N-Hydroxysuccinimidesters

(Z = 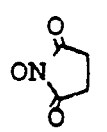 ),

diese wird mit der biologisch aktiven Substanz, bevorzugt $T_3$ oder $T_4$, in einem geeigneten Lösungsmittel wie DMF, gegebenenfalls unter Hinzufügung einer Base, wie Diisopropylamin, Triethylamin, 1,8-Diazabicyclo[5.4.0]undec-7-en oder 1,5-Diazobicyclo[4.3.0]non-5-en bei 0-50 °C, vorzugsweise bei Raumtemperatur, umgesetzt. Nach Abspaltung der Aminoschutzgruppe unter den für die gewählte Schutzgruppe geeigneten Bedingungen kann man nun

a) diese Verbindungen mit dem Protein, Oligo- oder Polypeptid umsetzen und anschließend dieses $[\text{Hapten-PEG}]_n$-P-Konjugat mit dem Label umsetzen, oder man stellt

b) zunächst ein Protein-$(\text{Label})_m$-Konjugat her und setzt dieses dann mit dem oben beschriebenen Hapten-PEG-Konjugat um.

Zur leichteren Umsetzung des Proteins mit dem Hapten-PEG-Konjugat empfiehlt es sich zunächst das Protein mit einem bifunktionellen Reagenz wie GMBS (γ-Maleinimidobuttersäure-N-succinimidylester) umzusetzen und das Konjugat ebenfalls, zwecks Generierung einer für die Umsetzung mit dem obengenannten Proteinderivat geeigneten funktionellen Gruppe, mit einem Reagenz zu versetzen. Ein geeignetes Reagenz für die letztgenannte Funktionalisierung ist beispielsweise SAMSA (S-Acetylmercaptobernsteinsäureanhydrid). Die so funktionalisierten Produkte werden dann im folgenden Reaktionsschritt miteinander umgesetzt, wobei $[\text{Hapten-PEG}]_n$-P-Konjugate entstehen. Die Umsetzung mit dem gewünschten Label, beispielsweise einem Acridinium-9-(N-sulfonyl)-carbonsäureamid, erfolgt vorzugsweise durch Umsetzung der beiden Ausgangsverbindungen (Label und Konjugat) in wäßriger Lösung, die gegebenenfalls auch Lösungsmittel wie DMF, DMSO, Acetonitril oder THF enthalten kann, bei einem pH-Wert von 6-9, bevorzugt 8, bei Raumtemperatur. Die Reaktionszeit beträgt 10-30 Minuten, bevorzugt 15 Minuten. Die Isolation und Reinigung kann beispielsweise durch Gelchromatographie oder durch Adsorption an Bio-beads erfolgen.

Die erfindungsgemäßen markierten Haptene eignen sich hervorragend für den Einsatz in Immunoassays zum Nachweis von Haptenen in Körperflüssigkeiten. Für einen solchen Immunoassay benötigt man einen gegen das zu bestimmende Hapten gerichteten Antikörper. Diese Antikörper poly- oder monoklonaler Art werden nach literaturbekannten Verfahren durch Immunisierung einer Spezies mit einem immunogenen Hapten-Konjugat des zu bestimmenden Haptens erhalten. Der so erhaltene Antikörper kann nun - ebenfalls nach literaturbekannten Verfahren - auf einer Festphase immobilisiert werden. Für die Durchführung des Immunoassays bieten sich verschiedene Möglichkeiten an. Eine bevorzugte Möglichkeit besteht darin, daß man den mit dem zu bestimmenden Hapten spezifisch reagierenden immobilisierten Antikörper mit einer Probe der zu untersuchenden Flüssigkeit, welche das zu bestimmende Hapten in unbekannter Konzentration enthält, und dem erfindungsgemäßen markierten Hapten inkubiert, die Probe und das nicht gebundene markierte Hapten abtrennt, und die Menge des gebundenen markierten Haptens unter Ausnutzung der speziellen chemischen oder physikalischen Eigenschaften des Labels bestimmt. Eine Variation dieses Immunoassays besteht darin, daß man zwischen Probenzugabe und Zugabe des markierten Haptens einen Trennschritt und gegegebenenfalls einen Waschschritt einführt.

Im folgenden wird die Erfindung anhand von Beispielen näher erläutert.

Beispiele

Beispiel 1

Ausgehend von PEG 600 ($\bar{x} \approx 13$) erhält man $T_3$-PEG-600-Poly (Glu:Lys 6:4)

Schritt 1:

74,4 g PEG 600 in 500 ml Toluol werden 4 Std. am Wasserabscheider gekocht, dann werden 10,8 ml Thionylchlorid und 10,8 ml Pyridin zugegeben und es wird weitere 4 Std. zum Rückfluß erhitzt. Nach Abkühlen und Abfiltrieren werden 200 ml $CH_2Cl_2$ zugegeben und es wird nochmals filtriert. Das Filtrat wird zur Trockene eingedampft, das Produkt fällt als öliger Rückstand an (76,7 g).

| Elementaraanalyse | | |
|---|---|---|
| ber.(als $Cl(CH_2CH_2O)_{13,2}H$): | C 51,3 % | Cl 5,74 |
| gef.: | C 50,4 % | Cl 5,7 |

Schritt 2:

61,8 g des Produkts aus Schritt 1 werden in 200 ml Diethylether gelöst, dazu gibt man 1,4 g $BF_3 \cdot (C_2H_5)_2O$ und 11,4 g $N_2CH$-$CO_2C_2H_5$ (Diazoessigsäureethylester). Es setzt eine lebhafte Gasentwicklung ein, man rührt 3 bis 5 Std. bei Raumtemperatur nach, bis die Gasentwicklung weitgehend abgeschlossen ist. Die Lösung wird mit $NaHCO_3$-Lösung gewaschen, die org. Phase z.B. über-$MgSO_4$ getrocknet und zur Trockene eingedampft (56 g).

Schritt 3:

5 g des Produkts aus Schritt 2 werden in 100 ml DMF 2 Std. mit 6,3 g Natriumazid auf 120 °C erhitzt. Das DMF wird im Vakuum abdestilliert, der Rückstand wird in 200 ml Wasser gelöst. Diese Lösung wird mit 4 N NaOH bei Raumtemperatur auf pH 11 eingestellt und bei diesem pH gehalten, bis keine NaOH mehr verbraucht wird. Nach Zugabe von festen NaCl wird die Lösung mit 6 N HCl auf pH 3 angesäuert und 3 x mit $CH_2Cl_2$ extrahiert. Die über $MgSO_4$ getrocknete Lösung wird eingeengt, man erhält 4,7 g eines öligen Produkts.

Schritt 4:

4,7 g des Produkts aus Schritt 3 werden in wenig Wasser gelöst und auf eine DEAE-Sephadex® A 25

(Pharmacia, 20 g, 70 mMol Anionenaustauscherkapazität) in der Tetraboratform enthaltende Säule gegeben. Mit Wasser eluiert man eine neutrale Fraktion (vorwiegend PEG-Diazid, Formel VIII, y = 2, z = 0), bei schrittweiser Erhöhung des $NH_4HCO_3$-Gehalts (beginnend bei 4 mM in 3 mM Schritten) erhält man zunächst eine Fraktion mit der gewünschten PEG-Azidcarbonsäure (Formel VIII, y = z = 1), bei weiterer Erhöhung der $NH_4HCO_3$-Konzentration wird auch die PEG-Dicarbonsäure (Formel VIII, y = 0, z = 2) eluiert. Aus der mittleren Fraktion gewinnt man die PEG-Azidcarbonsäure durch Einengen der Lösung zur Trockene, Verrühren des Rückstands mit $CH_2Cl_2$, Abfiltrieren des unlöslichen Anteils, Trocknen der org. Phase über $MgSO_4$ und Abdampfen des Lösungsmittels (1,4 g).

Die Reaktion und Trennung können durch Dünnschichtchromatographie auf $SiO_2$ mit Isopropanol : konz. $NH_3$ : $H_2O$ 10 : 2 : 1 als Laufmittel und $I_2$ als Nachweismethode verfolgt werden. Die gewünschte PEG-Azidcarbonsäure hat einen $R_f$-Wert von ~0,5 (PEG-Diazid: $R_f$ = 0,75, PEG-Dicarbonsäure $R_f$ = 0,35).

IR : 3500 $cm^{-1}$ (br) OH
: 2120 $cm^{-1}$ $N_3$ (fehlt bei 3. Frakt.)
: 1700 $cm^{-1}$

O
‖
C

(fehlt bei 1. Frakt.)
: 1120 $cm^{-1}$ -O-

Schritt 5:

1,4 g der in Schritt 4 erhaltenen PEG-Azidcarbonsäure werden in 20 ml einer 4:1 Mischung von-Ethanol und $CH_2Cl_2$ gelöst und mit Pd/C-Katalysator bei Normaldruck hydriert. Nach beendeter Hydrierung wird der Katalysator abgesaugt, das Filtrat wird eingeengt. Der Rückstand wird in 25 ml 2 N NaOH gelöst und mit 8,7 ml Ditert.-butyldicarbonat bei Raumtemperatur 20 Std. gerührt. Die Mischung wird mit Hexan extrahiert, die wäßrige Phase anschließend mit gesätt. $KHSO_4$ Lösung auf pH 3 angesäuert und mit $CH_2Cl_2$ 3 x extrahiert. Aus der über $MgSO_4$ getrockneten vereinigten org. Phase erhält man durch Eindampfen 1,3 g tert.-Butyloxycarbonylamino-PEG-600-carbonsäure.

Schritt 6:

1 g Produkt aus Schritt 5 in 7 ml THF werden bei -20 °C mit 115 mg N-Hydroxysuccinimid und 280 mg Dicyclohexylcarbodiimid 1 Std. gerührt, danach läßt man auftauen und 1 Std. bei Raumtemperatur nachrühren. Der entstandene Niederschlag wird abgesaugt, das Filtrat eingeengt, der Rückstand in kaltem Aceton aufgenommen, evtl. ausfallender Niederschlag wird abgesaugt, das Filtrat wird zur Trockene eingedampft. Der Rückstand wird in wenig DMF aufgenommen und zu einer Lösung aus 840 mg $T_3$-hydrochlorid und 340 μl Triethylamin in 10 ml DMF gegeben. Man läßt 24 Std. bei Raumtemperatur rühren, dampft das DMF im Vakuum ab und erhält das gewünschte Produkt als zähes, glasartig erstarrendes Öl.

Die Reinigung des Rohprodukts kann durch Mitteldruckchromatographie an RP-18 mit MeOH : $H_2O$ 77 : 23 + 0,1 Vol-% Trifluoressigsäure erfolgen.

Das gereinigte Produkt (1 g) wird in 10 ml Trifluoressigsäure aufgenommen und 1 Std. im Eisbad gerührt. Die Trifluoressigsäure wird abgezogen, das

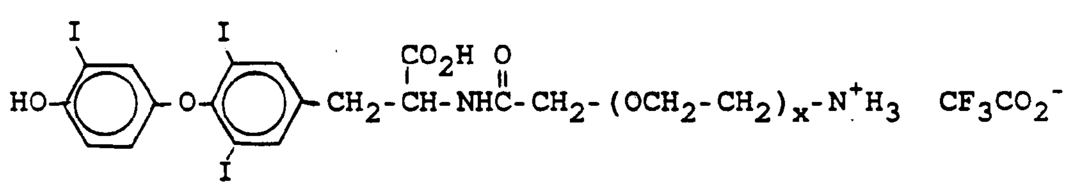

fällt als zähes Öl an.

Massenspektrum: FAB, 3-Nitrobenzylalkohol-Matrix

| $MH^+$ | rel. Intens. [%] | Kettenlänge X |
|---|---|---|
| 1017 | 5 | 7 |
| 1061 | 14 | 8 |
| 1105 | 30 | 9 |
| 1149 | 52 | 10 |
| 1193 | 79 | 11 |
| 1237 | 100 | 12 |
| 1281 | 94 | 13 |
| 1325 | 78 | 14 |
| 1369 | 59 | 15 |
| 1413 | 36 | 16 |
| 1457 | 22 | 17 |
| 1501 | 13 | 18 |
| 1545 | 4 | 19 |

Schritt 7:

a) 10 mg Poly (Glu:Lys 6:4) (Sigma) in 3,75 ml 0,1 M Natriumborat-Puffer (pH 8,2) werden mit 240 µl Dioxan und 112 µl einer GMBS (γ-Maleinimidobuttersäure-N-succinimidylester)-Lösung (10 mg GMBS/ml Dioxan) 1 Std. bei Raumtemperatur umgesetzt und anschließend 2 Std. gegen Wasser dialysiert.
b) 10,8 mg des Produkts aus Schritt 6 in 500 µl DMSO werden mit 25 µl N-Ethylmorpholin und 64 µl einer SAMSA (S-Acetylmercaptobernsteinsäureanhydrid)-Lösung (20 mg SAMSA/ml DMSO) 30 min unter Lichtausschluß bei Raumtemperatur umgesetzt, anschließend setzt man 71,5 µl einer 1 M Hydroxylaminhydrochlorid-Lösung zu und läßt 15 min im Dunkeln stehen.
c) Das Dialysat aus a) wird mit 375 µl DMSO und 250 µl der Lösung aus b) 1 Std. im Dunkeln bei Raumtemperatur umgesetzt, danach wird 1 µl Mercaptopropionsäure zugegeben und nach 10 min im Dunkeln wird 3 Std. gegen 10 mM Phosphatpuffer (pH 8,0) dialysiert. Das Dialysat wird über einen 0,45 µm Filter filtriert und eingefroren.

Schritt 8:

In einer 3 ml Rollrandflasche werden 600 µl Konjugationspuffer (0,45 g $KH_2PO_4$, 8,32 g $Na_2HPO_4 \cdot 2\,H_2O$, 4,36 g NaCl in 0,5 1 demineralisiertes $H_2O$; mit NaOH auf pH 8 gestellt) vorgelegt. Dazu gibt man 100 µl des Dialysats aus Schritt 7c und 20 µg N(4-Methoxyphenyl)-N-[4-(2-Succinimidyloxycarbonylethyl)-benzolsulfonyl]-10-methylacridinium-9-carbonsäureamid-fluorosulfonat (Beispiel 1 der EP 330 050) in Form einer Acetonitrillösung (1 mg/ml). Dann wird 15 Minuten im Dunkeln bei Raumtemperatur stehengelassen und anschließend 200 µl L-Lysinlösung (10 mg/ml Konjugationspuffer) hinzugegeben und weitere 15 Minuten im Dunkeln inkubiert. Die resultierende Lösung wird dann über eine mit 20 ml Elutionspuffer (4,08 g $KH_2PO_4$, 3,56 g $Na_2HPO_4 \cdot 2\,H_2O$, 4,38 g NaCl, 0,25 g $NaN_3$, in 0,5 l demineralisiertem $H_2O$; mit HCl auf pH 6,3 gestellt) equilibrierte PD-10®-Säule (Pharmacia) gereinigt.

Beispiel 2

Ausgehend von PEG 400 ($\bar{x} \approx 8{,}7$) erhält man analog zu Beispiel 1 $T_3$-PEG-400-Poly (Glu:Lys 6:4)

Schritt 1:

Elementaranalyse

| | | |
|---|---|---|
| ber. (als $Cl(CH_2-CH_2O)_{8,7}H$): | C: 49,78 | Cl 8,46 |
| gef.: | C: 49,3 | Cl 8,8 |

Schritt 6:

Massenspektrum: FAB, 3-Nitrobenzylalkohol-Matrix

| $MH^+$ | rel. Intens. [%] | Kettenlänge X |
|---|---|---|
| 841 | 9 | 3 |
| 885 | 17 | 4 |
| 929 | 29 | 5 |
| 973 | 68 | 6 |
| 1017 | 100 | 7 |
| 1061 | 95 | 8 |
| 1105 | 70 | 9 |
| 1149 | 50 | 10 |
| 1193 | 26 | 11 |
| 1237 | 12 | 12 |

Schritt 7b:

Es wurden 9,25 mg des Produkts aus Schritt 6 verwendet und wie in Schritt 7b und c des Beispiels 1 verfahren.

Schritt 8:

In einer 3 ml Rollrandflasche werden 600 µl Konjugationspuffer (0,45 g $KH_2PO_4$, 8,32 g $Na_2HPO_4 \cdot 2\,H_2O$, 4,36 g NaCl in 0,5 l demineralisiertes $H_2O$; mit NaOH auf pH 8 gestellt) vorgelegt. Dazu gibt man 100 µl des Dialysats aus Schritt 7c und 20 µg N-(4-Methoxyphenyl)-N-[4-(2-Succinimidyloxycarbonylethyl)-benzolsulfonyl]-10-methylacridinium-9-carbonsäureamid-fluorosulfonat (Beispiel 1 der EP 330 050) in Form einer Acetonitrillösung (1 mg/ml). Dann wird 15 Minuten im Dunkeln bei Raumtemperatur stehengelassen und anschließend 200 µl L-Lysinlösung (10 mg/ml Konjugationspuffer) hinzugegeben und weitere 15 Minuten im Dunkeln inkubiert. Die resultierende Lösung wird dann über eine mit 20 ml Elutionspuffer (4,08 g $KH_2PO_4$, 3,56 g $Na_2HPO_4 \cdot 2\,H_2O$, 4,38 g NaCl, 0,25 g $NaN_3$, in 0,5 l demineralisiertem $H_2O$; mit HCl auf pH 6,3 gestellt) equilibrierte PD-10-Säule (Pharmacia) gereinigt.

Beispiel 3

Triethylenglykolmonochlorhydrin ist käuflich, daher kann auf den Schritt 1 (partielle Chlorierung) und den Schritt 4 (Trennung der Mischung aus PEG-Diazid, PEG-Azidcarbonsäure und PEG-Dicarbonsäure) verzichtet werden. Die restlichen Schritte verlaufen analog zu den oben Beschriebenen.

Schritt 6:

Massenspektrum: FAB, 3-Nitrobenzylalkohol-Matrix

| $MH^+$ | rel. Intens. [%] | Kettenlänge X |
|---|---|---|
| 753 | 1 | 1 |
| 797 | 7 | 2 |
| 841 | 100 | 3 |
| 885 | 3 | 4 |
| 929 | 1 | 5 |

Schritt 7b:

7,1 mg des Produkts aus Schritt 6 werden wie in Schritt 7b und c des Beispiels 1 umgesetzt.

Schritt 8:

In einer 3 ml Rollrandflasche werden 600 µl Konjugationspuffer (0,45 g $KH_2PO_4$, 8,32 g $Na_2HPO_4 \cdot 2 H_2O$, 4,36 g NaCl in 0,5 l demineralisiertes $H_2O$; mit NaOH auf pH 8 gestellt) vorgelegt. Dazu gibt man 100 µl des Dialysats aus Schritt 7c und 20 µg N-(4-Methoxyphenyl)-N-[4-(2-Succinimidyloxycarbonylethyl)-benzolsulfonyl]-10-methylacridinium-9-carbonsäureamid-fluorosulfonat (Beispiel 1 der EP 330 050) in Form einer Acetonitrillösung (1 mg/ml). Dann wird 15 Minuten im Dunkeln bei Raumtemperatur stehengelassen und anschließend 200 µl L-Lysinlösung (10 mg/ml Konjugationspuffer) hinzugegeben und weitere 15 Minuten im Dunkeln inkubiert. Die resultierende Lösung wird dann über eine mit 20 ml Elutionspuffer (4,08 g $KH_2PO_4$, 3,56 g $Na_2HPO_4 \cdot 2 H_2O$, 4,38 g NaCl, 0,25 g $NaN_3$, in 0,5 l demineralisiertem $H_2O$; mit HCl auf pH 6,3 gestellt) equilibrierte PD-10-Säule (Pharmacia) gereinigt.

Beispiel 4

Ausgehend von PEG 2000 wurde exakt nach Polym. Prep. <u>27</u>, 1 (1986)

$$t\text{-}BOC\text{-}NH\text{-}(CH_2\text{-}CH_2O)_x\overset{\overset{O}{\|}}{C}\text{-}NHCH_2CO_2H$$

dargestellt. Schritt 6 erfolgt analog zu Beispiel 1

Schritt 6:

Massenspektrum: FAB, 3-Nitrobenzylalkohol-Matrix

| $MH^+$ | rel. Intens. [%] | Kettenlänge X |
|---|---|---|
| 2426 | 14 | 38 |
| 2470 | 20 | 39 |
| 2514 | 30 | 40 |
| 2558 | 47 | 41 |
| 2602 | 67 | 42 |
| 2646 | 83 | 43 |
| 2690 | 95 | 44 |

| $MH^+$ | rel. Intens. [%] | Kettenlänge X |
|---|---|---|
| 2734 | 100 | 45 |
| 2778 | 99 | 46 |
| 2822 | 93 | 47 |
| 2866 | 84 | 48 |
| 2910 | 74 | 49 |
| 2954 | 62 | 50 |
| 2998 | 51 | 51 |
| 3042 | 40 | 52 |
| 3086 | 31 | 53 |
| 3130 | 23 | 54 |
| 3174 | 15 | 55 |
| 3218 | 9 | 56 |
| 3262 | 5 | 57 |

Schritt 8:

In einer 3 ml Rollrandflasche werden 600 µl Konjugationspuffer (0,45 g $KH_2PO_4$, 8,32 g $Na_2HPO_4 \cdot 2\ H_2O$, 4,36 g NaCl in 0,5 l demineralisiertes $H_2O$; mit NaOH auf pH 8 gestellt) vorgelegt. Dazu gibt man 100 µl des Dialysats aus Schritt 7c und 20 µg N-(4-Methoxyphenyl)-N-[4-(2-Succinimidyloxycarbonylethyl)-benzolsulfonyl]-10-methylacridinium-9-carbonsäureamid-fluorosulfonat (Beispiel 1 der EP 330 050) in Form einer Acetonitrillösung (1 mg/ml). Dann wird 15 Minuten im Dunkeln bei Raumtemperatur stehengelassen und anschließend 200 µl L-Lysinlösung (10 mg/ml Konjugationspuffer) hinzugegeben und weitere 15 Minuten im Dunkeln inkubiert. Die resultierende Lösung wird dann über eine mit 20 ml Elutionspuffer (4,08 g $KH_2PO_4$, 3,56 g $Na_2HPO_4 \cdot 2\ H_2O$, 4,38 g NaCl, 0,25 g $NaN_3$, in 0,5 l demineralisiertem $H_2O$; mit HCl auf pH 6,3 gestellt) equilibrierte PD-10-Säule (Pharmacia) gereinigt.

Beispiel 5

Ausgehend von PEG 2000 wurde exakt nach Polym. Prep. 27, 1 (1986)

$$t\text{-}BOC\text{-}NH\text{-}(CH_2\text{-}CH_2O)_X\text{-}\overset{O}{\overset{\|}{C}}NH\text{-}CH_2CO_2H$$

dargestellt.

Schritt 6:

2,2 g diese Produkts in 15 ml THF + 15 ml DMF werden bei -20 °C mit 120 mg N-Hydroxysuccinimid und 226 mg Dicyclohexylcarbodiimid 2 Std. gerührt, man läßt auftauen und rührt bei Raumtemperatur nach. Ausgefallener Niederschlag wird abfiltriert, das Filtrat eingedampft, der Rückstand wird in kaltem Aceton aufgenommen, ausfallender Niederschlag wird abgesaugt. Der nach dem Eindampfen des Filtrats verbleibende Rückstand wird in 10 ml DMF aufgenommen und zu einer Lösung von 770 mg L- $T_4$ und 276 µl Triethylamin in 20 ml DMF gegeben und 24 Std. bei Raumtemperatur gerührt. Das Lösungsmittel wird abgezogen und das Rohprodukt wie in Beispiel 1 gereinigt. Zur Entfernung der Schutzgruppe wird die Substanz in 10 ml Trifluoressigsäure gelöst und 1 Std. bei 0 °C gerührt, anschließend wird die Trifluoressigsäure abgezogen und man erhält

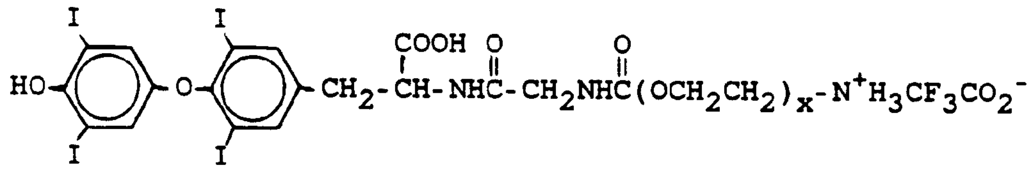

als glasartig erstarrendes Öl.

Massenspektrum: FAB, 3-Nitrobenzylalkohol-Matrix

| $MH^+$ | rel. Intens. [%] | Kettenlänge X |
|---|---|---|
| 2552 | 25 | 38 |
| 2596 | 27 | 39 |
| 2640 | 35 | 40 |
| 2684 | 47 | 41 |
| 2728 | 62 | 42 |
| 2772 | 79 | 43 |
| 2816 | 88 | 44 |
| 2860 | 93 | 45 |
| 2904 | 100 | 46 |

| $MH^+$ | rel. Intens. [%] | Kettenlänge X |
|---|---|---|
| 2948 | 97 | 47 |
| 2992 | 87 | 48 |
| 3036 | 82 | 49 |
| 3080 | 73 | 50 |
| 3124 | 64 | 51 |
| 3168 | 53 | 52 |
| 3212 | 45 | 53 |
| 3256 | 32 | 54 |
| 3300 | 23 | 55 |

Schritt 8:

In einer 3 ml Rollrandflasche werden 600 µl Konjugationspuffer (0,45 g $KH_2PO_4$, 8,32 g $Na_2HPO_4 \cdot 2\ H_2O$, 4,36 g NaCl in 0,5 l demineralisiertes $H_2O$; mit NaOH auf pH 8 gestellt) vorgelegt. Dazu gibt man 100 µl des Dialysats aus Schritt 7c und 20 µg N-(4-Methoxyphenyl)-N-[4-(2-Succinimidyloxycarbonylethyl)-benzolsulfonyl]-10-methylacridinium-9-carbonsäureamid-fluorosulfonat (Beispiel 1 der EP 330 050) in Form einer Acetonitrillösung (1 mg/ml). Dann wird 15 Minuten im Dunkeln bei Raumtemperatur stehengelassen und anschließend 200 µl L-Lysinlösung (10 mg/ml Konjugationspuffer) hinzugegeben und weitere 15 Minuten im Dunkeln inkubiert. Die resultierende Lösung wird dann über eine mit 20 ml Elutionspuffer (4,08 g $KH_2PO_4$, 3,56 g $Na_2HPO_4 \cdot 2\ H_2O$, 4,38 g NaCl, 0,25 g $NaN_3$, in 0,5 l demineralisiertem $H_2O$; mit HCl auf pH 6,3 gestellt) equilibrierte PD-10-Säule (Pharmacia) gereinigt.

Die Synthese der Verbindungen aus den folgenden Beispielen ist in Syntheseschema 2 dargestellt.

**Carbonsäureanhydrid-Diamin-Addukte 3** (Schritt 1)

Zu einer Lösung von 45 mmol (9,19 g; 9,56 ml) 1,12-Diamino-4,9-dioxadodecan in 400 ml trockenem Acetonitril tropfte man bei 0°C unter Rühren langsam (~4 h) eine Lösung von 40 mmol Bernsteinsäureanhydrid (4,00 g) bzw. Diglycolsäureanhydrid (4,64 g) in 200 ml trocknem Acetonitril. Einige Minuten nach Beginn des Zutropfens bildete sich ein farbloser Niederschlag.

Nach Beendigung des Zutropfens rührte man die Suspension noch 2 - 3 Stunden bei Raumtemperatur, kühlte im Eisbad ab, gab 200 ml Diethylether zu, filtrierte den Niederschlag über eine Umkehrfritte unter Stickstoff ab (Feuchtigkeitsausschluß!), wusch auf der Fritte zunächst mit Acetonitril, dann mit Diethylether und trocknete den farblosen Niederschlag im Vakuum.

Ausbeuten 95 % - quantitativ

$C_{14}H_{28}N_2O_5$ (304.4)

3a: 12,08 g (99 %); Schmp. 124 - 126°C;

IR (KBr): 3320 (N-H); 3060 (br,-$N^+H_3$); 2950, 2865, 2805 (C-H); 2500, 2140 (br., -$N^+H_3$); 1640 (C=O, Amid I); 1595 (C=O, -$CO_2^-$); 1560, 1540 (Amid II); 1120 (-C-O-C-).

$^1$H-NMR (100 MHz, $D_2O$): $\delta$ = 1.5 - 2.1 (m, 8H); 2.44 (s, 4H); 3.10 (t, 2H; C-$CH_2$-NH); 3.23 (t, 2H; -$CH_2N^+H_3$); 3.39 - 3.70 (m, 8H, -$CH_2$-O-$CH_2$-);

MS (FAB): $\frac{m}{z}$ (%) = 305 (100; $MH^+$)

3b: $C_{14}H_{28}N_2O_6$ (320,4); 12,69 g (99 %); Schmp. 88 - 91°C;

IR (KBr): 3370 (N-H); 3060 (br., -$N^+H_3$); 2950, 2860, 2805 (C-H); 2550, 2140 (br., -$N^+H_3$); 1660 (C=O, Amid I); 1595 (C=O, -$CO_2^-$); 1560, 1545 (Amid II); 1125 (-C-O-C-).

$^1$H-NMR (100 MHz, $D_2O$): $\delta$ = 1.50 - 2.08 (m, 8H); 3.08 (t, 2H, C-$CH_2$-N-H): 3.34 (t, 2H; -$CH_2$-$N^+H_3$); 3.44 - 3.69 (m, 8H; -$CH_2$-O-$CH_2$); 3.99 (s, 2H); 4.06 (s, 2H).

**N-t-Boc-Systeme 4** (Schritt 2)

(allgemeine Vorschrift zur Einführung von t-Boc-Schutzgruppen)

13 mmol (3,96 g) 3a wurden in 45 ml eines Dioxan/Wasser-Gemisches (2:1) gelöst. Nach Zugabe von 15 ml 1 M Natronlauge wurden unter Kühlung im Eisbad 26 mmol (5,66 g) Di-tert.-butyldicarbonat zugesetzt und 10 Minuten bei 0°C gerührt, dann ließ man auf Raumtemperatur erwärmen und rührte noch 4 Stunden. Nach weitgehendem Abdestillieren des Dioxans im Vakuum überschichtete man den Rückstand mit Essigester und säuerte, unter kräftigem Rühren und Kühlung im Eisbad, mit gesättigter Kaliumhydrogensulfat-Lösung auf einen pH-Wert von ~3 an.

Nach Abtrennen der organischen Phase extrahiert man die Wasserphase viermal mit Essigester, wusch die vereinigten organischen Phasen mit Wasser und trocknete mit Natriumsulfat. Nach Abdestillieren des Lösungsmittels im Vakuum erhielt man 8,2 g eines schwach gelben Öls, das in 15 ml Diethylether aufgenommen wurde. Bei 0°C tropfte man unter Rühren n-Hexan zu, bis sich ein farbloser Niederschlag bildete. Nach 2 Stunden Stehen im Eisbad filtrierte man den Niederschlag ab, wusch gründlich mit Hexan und trocknete im Vakuum. Man erhielt 3,73 g (71 %) eines farblosen Feststoffs.

4a: $C_{19}H_{36}N_2O_7$ (404.5), Schmp. 44 - 46°C

IR (KBr): 3360, 3330 (N-H); 3200 - 2500

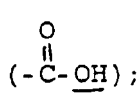

2940, 2860, 2805 (C-H); 1690

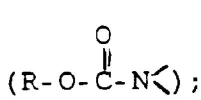

1640 (C=O, Amid I); 1535 (Amid II); 1385, 1365 ($C(CH_3)_3$); 1115 (-C-O-C-).

$^1$H-NMR (100 MHz; $CDCl_3$): $\delta$ = 1.44 (s, 9H, tBu); 1.55 - 1.90 (m, 8H, -C-$CH_2$-C-); 2.36 - 2.78

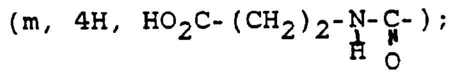

3.08 - 3.59 (m, 12H, $-O-CH_2-$, $-N-CH_2-$); 4.99, 6.70, 8,48 (breit, jeweils 1H, N-H, $CO_2H$).

Aus 10 mmol (3,20 g) 3b und 20 mmol (4,37 g) Di-tert.-butyldicarbonat erhielt man nach obiger Vorschrift als Rohprodukt 5,17 g gelbes Öl. Es gelang nicht wie oben einen Niederschlag zu bilden. Zur Entfernung von überschüssigem Di-tert.-butyldicarbonat wurde das Öl in 400 ml Wasser aufgenommen. Man extrahierte viermal mit Hexan, sättigte dann die Wasserphase mit Natriumchlorid und extrahierte fünfmal mit Essigester. Nach Trocknen der Essigester-Extrakte mit Natriumsulfat und Abdestillieren des Lösungsmittels im Vakuum (letzte Lösungsmittelreste wurden bei ~0.1 mbar entfernt) erhielt man 3,08 g (73 %) eines fast farblosen Öls.

4b: $C_{19}H_{36}N_2O_8$ (420.4)

IR (Film): 3340 (N-H); 3200 - 2400 ($-CO_2H$); 2940, 2865 (C-H); 1720

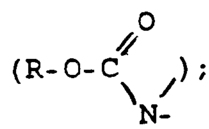

1665 (C=O, Amid I); 1540 (Amid II); 1390, 1365 ($C(CH_3)_3$); 1140 (-C-O-C-).

$^1$H-NMR (100 MHz, $CDCl_3$): $\delta$ = 1.45 (9H,t-Bu); 1.55 - 1.95 (m, 9H, $-C-CH_2-C-$); 3.08 - 3.62 (m, 12H, $-O-CH_2$, $-N-CH_2-$); 4.10 (s, 2H); 4.15 (s, 2H); 5.00, 7.54, 8.35 (breit, jeweils 1H; N-H, $-CO_2H$).

**"Doppel-Spacer" 5** (Schritt 3; nur für Beispiel 8,9)

5,0 mmol (2,02 g) 4a und 5,5 mmol (633 mg) N-Hydroxysuccinimid wurden in 40 ml Tetrahydrofuran gelöst, die Lösung auf -20°C abgekühlt, mit 5,5 mmol (1,13 g) Dicyclohexylcarbodiimid versetzt, 2 Stunden bei -20°C gerührt. Dann ließ man auf Raumtemperatur erwärmen, destillierte das Lösungsmittel im Vakuum ab, digerierte das verbliebene Öl mit eiskaltem Aceton und filtrierte sofort den farblosen Niederschlag (Dicyclohexylharnstoff) ab. Dieser Vorgang wurde noch zweimal wiederholt. Dann destillierte man das Lösungsmittel im Vakuum ab (zuletzt bei ~0.1 mbar), löste das verbleibende Öl (2,70 g) in 30 ml Chloroform, gab 100 mmol (1,01 g, 1,39 ml) Triethylamin und 50 mmol 3a zu und rührte die Mischung bei Raumtemperatur. Nach wenigen Minuten war eine klare Lösung entstanden, die man 40 Stunden bei Raumtemperatur stehen ließ. Dann wurde das Lösungsmittel im Vakuum abdestilliert, der ölige Rückstand mit Wasser versetzt und mit gesättigter Kaliumhydrogensulfat-Lösung auf pH ~3 angesäuert. Nach Sättigen mit Natriumchlorid extrahierte man fünfmal mit Essigester, wusch die vereinigten Extrakte mit gesättigter Natriumchloridlösung, trocknete mit Natriumsulfat und destillierte das Lösungsmittel im Vakuum ab. Man erhielt nach Trocknen im Vakuumexsikkator bei ~0.1 mbar 3,21 g (93 %) eines farblosen Pulvers, das für weitere Umsetzungen ausreichend rein war.

5a: $C_{33}H_{62}N_4O_{11}$ (690.9)

IR (KBr): 3360, 3320, 3070 (N-H); 2950, 2870, 2810 (C-H); 3200 - 2500 (COOH); 1720, 1695, 1690

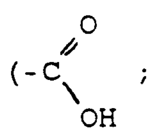

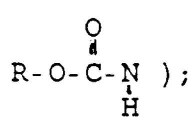

1640, 1630 (C=O, Amid); 1540 (Amid II); 1370 (t-Bu); 1140 (-C-O-C-).

$^1$H-NMR (100 MHz; $CDCl_3$): δ = 1.43 (s, 9H, t-Bu); 1.53 - 1.89 (m, 16H, $-C-CH_2-C-$); 2.50 (s, 4H,

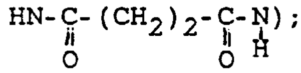

2.43 - 2.74 (m, 4H,

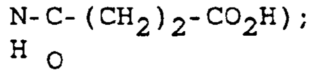

3.06 - 3.59 (m, 24H, $-CH_2-O$, $-CH_2-N$); 5.00, 6.68 - 7.09 (breit, N-H, $CO_2H$).

MS (FAB): m/z (%) = 713 (55) [$MNa^+$]; 691 (44) [$MH^+$]; 591 (100)

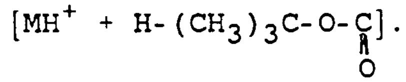

Wie oben beschrieben erhielt man aus 2,38 mmol (1,00 g) 4b und 2,38 mmol (763 mg) 3b 1,36 g (79 %) eines schwach gelben Öls.

5b: $C_{33}H_{62}N_4O_{13}$ (722.8)

$^1$H-NMR (100 MHz, $CDCl_3$): δ = 1.43 (s, 9H, tBu); 1.53 - 1.95 (m, 16H, $-C-CH_2-C-$); 3.05 - 3.60 (m, 24H, $-CH_2-O-$, $-CH_2-NH$); 4.05, 4.08, 4.16

(alle s, 8H, 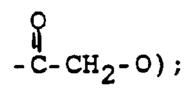);

5.05, 7.30, 7.58 (alle breit, -NH, $-CO_2H$).

**Ankoppeln von $T_3$** (Schritt 4) und **Schutzgruppenabspaltung** (Schritt 5)

In Analogie zu Beispiel 1, Schritt 6, wurden 809 mg (2mmol) 4a mit N-Hydroxysuccinimid/Dicyclohexylcarbodiimid umgesetzt. Der erhaltene N-Hydroxysuccinimidester wurde mit 1,37 g (2 mmol) $T_3$-hydrochlorid und 555 µl (4 mmol) Triethylamin in 10 ml Dimethylformamid 24 h bei Raumtemperatur gerührt. Nach Abfiltrieren eines farblosen Niederschlages wurde die Lösung mit Wasser versetzt, mit Kaliumhydrogensulfat-Lösung auf pH ~3 angesäuert und nach Sättigen mit Natriumchlorid fünfmal mit Essigester extrahiert. Nach Waschen der vereinigten Essigester-Extrakte mit Natriumchlorid-Lösung, Trocknen mit Natriumsulfat und Abdestillieren des Lösungsmittels erhielt man ein schwachgelbes Pulver. Die Reinigung des Produktes erfolgte durch Mitteldruckchromatographie an RP-18 mit Methanol:$H_2O$ = 73:27 + 0,1 Vol.-% Trifluoressigsäure.

1,0 g des Produktes 6a wurde analog Beispiel 1 mit Trifluoressigsäure behandelt. Das erhaltene ölige Produkt ergab durch Behandeln mit Diethylether ein farbloses Pulver, das abfiltriert und im Vakuum getrocknet wurde.

6a: $C_{34}H_{46}I_3N_3O_{10}$ (1037.4)

MS (FAB, 3-Nitrobenzylalkohol-Matrix):

m/z (%) = 1060 (6) $MNa^+$, 1038 (15) $MH^+$

7a:

$$[C_{29}H_{39}I_3N_3O_8]^+[C_2F_3O_2]^-$$
$$(938.3) \quad (113.0)$$

MS (FAB, 3-Nitrobenzylalkohol-Matrix):

m/z (%) = 938 (100) $M^+$

IR (KBr): 3400, 3260

(N-H); 3060 $(-N^+H_3)$;
O-H -NH

3200-2400 ($-CO_2H$); 2930, 2860, 2810 (C-H); 1730, 1720

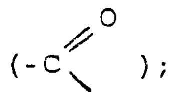

1670, 1650

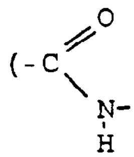
, Amid I);

1540 (Amid II); 1200, 1175, 1130.

$^1$H-NMR (360-MHz, $[D_6]$-DMSO):

δ = 1.51 (m, 4H, O-C-$(\underline{CH_2})_2$-C-O); 1.59 (m, 2H, -$\underline{CH_2}$-$CH_2$-$N^+H_3$); 1.77 (m, 2H, N(H)-C-$\underline{CH_2}$-C-O-);

2.25, 2.30 (2m, 4H, N(H)-C(=O)-$(\underline{CH_2})_2$-C(=O)-N(H));

2.82 (m, 3H, -$CH_2$-$\underline{CH_2}$-$N^+H_3$, Ar-$\underline{CH_2}$-C(H)($CO_2H$)-NH);

3.08 (m, 3H, -C(=O)-N(H)-$\underline{CH_2}$, Ar-$\underline{CH_2}$-C(H)($CO_2H$)-NH);

3.25-3.43 (m, 8H, N-$CH_2$-, O-$CH_2$); 4.42 (m, 1H, -N(H)-$\underline{CH}$($CO_2H$)-$CH_2$-); 6.56 (dd, 1H, Aryl-$H_b$); 6.82 (d, 1H, Aryl-$H_c$); 6.98 (d, 1H, Aryl $H_a$); 7.65 (breit, 3H,$N^+H_3$); 7.69 (m, 1H, C(=O)-N($\underline{H}$)-$CH_2$-$CH_2$);

7.78 (s, 2H, Aryl-$H_d$); 8.19 (d, 1H, $CH_2$-CH($CO_2H$)-N($\underline{H}$)-C(=O)-);

9.96 (breit, 1H, Ar-O$\underline{H}$); 12.70 (breit, 1H,-$CO_2H$).

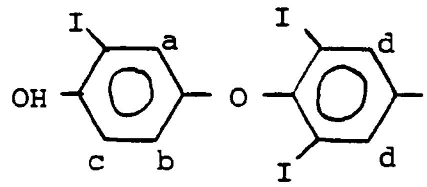

6b, 7b:

Aus 4b und $T_3$-Hydrochlorid erhielt man analog obiger Vorschrift 6b und 7b.

6b: $C_{34}H_{46}I_3N_3O_{11}$ (1053.4)

MS (FAB, 3-Nitrobenzylalkohol-Matrix mit und ohne KCl-Zusatz):

m/z (%) = 1092 (18) [$MK^+$], 1053 (4) [$M^+$], 954 (85) [M-$(CH_3)_3$OCO + 2H].

7b:

$$[C_{29}H_{39}I_3N_3O_9]^+ \quad [C_2F_3O_2]^-$$
$$(954.3) \qquad (113.0)$$

MS (FAB, 3-Nitrobenzylalkohol-Matrix):
m/z (%) = 954 (20) [$M^+$]

IR (KBr): 3400, 3260 (O-H, N-H); 3070 (-$N^+H_3$, -NH-); 3200-2400 (-$CO_2H$); 1730, 1710

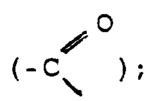

1670, 1650

$(-\overset{O}{\overset{\|}{C}}-\overset{H}{\overset{|}{N}}-$, Amid I);

1540 (Amid II); 1200, 1170, 1125.

$^1$H-NMR (360-MHz, $D_6$-DMSO):

δ = 1.52 (m, 4H, O-$CH_2$-($\underline{CH_2}$)$_2$-$CH_2$-O); 1.68 (m, 2H, -$CH_2$-$\underline{CH_2}$-$CH_2$-$N^+H_3$); 1.77 (m, 2H, -$\underset{H}{N}$-$CH_2$-$\underline{CH_2}$-$CH_2$-O); 2.73-2.97 (m, 3H, -$\underline{CH_2}$-$N^+H_3$, Ar-$\underline{CH_2}$-$\underset{CO_2H}{CH}$-NH-); 3.07-3.23 (m, 3H, -$\underset{O}{\overset{\|}{C}}$-$\underset{H}{N}$-$\underline{CH_2}$-$CH_2$-, Ar-$\underline{CH_2}$-$\underset{CO_2H}{CH}$-NH-); 3.23-3.48 (m, 8H, O-$\underline{CH_2}$-$CH_2$-); 3.80-4.03 (m, 4H, -$\overset{O}{\overset{\|}{C}}$-$\underline{CH_2}$-O-$\underline{CH_2}$-$\overset{O}{\overset{\|}{C}}$-); 4.53 (m, 1H, -$CH_2$-$\overset{CO_2H}{\underline{CH}}$-NH); 6.56 (dd, 1H, Aryl-$H_b$); 6.82 (d, 1H, Aryl-$H_c$); 6.97 (d, 1H, Aryl-$H_a$);

7.65 (breit, 3H, $-N^{+}H_3$); 7.78 (s, 2H, Aryl-$H_d$);

7.95 (m, 1H, 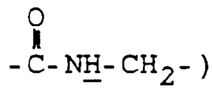); 8.32 (d, 1H,

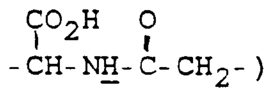); 9.98 (breit, 1H, Ar-<u>OH</u>);

12.85 (breit, 1H, $-CO_2H$).

<u>6c</u>, <u>7c</u>

Aus <u>5a</u> und $T_3$-Hydrochlorid analog obiger Vorschrift

<u>6c</u>: $C_{48}H_{72}I_3N_5O_{14}$ (1323.8)

MS (FAB, 3-Nitrobenzylalkohol-Matrix): m/z (%) = 1324 (55) $MH^+$, 1224 (100) [$M^+$-$(CH_3)_3C$-O-CO + 2H].

IR (KBr): 3300 (O-H, N-H); 3080 (N-H, Amid); 3200-2400 ($-CO_2H$); 2940, 2860, 2800 (C-H); 1735, 1720

(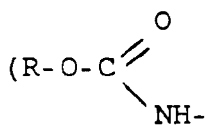, 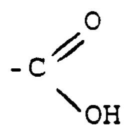); 1650 (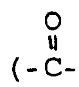, Amid I);

1540 (Amid II); 1365 ($C(CH_3)_3$); 1110 (-C-O-C-).

$^1$H-NMR (360-MHz, $D_6$-DMSO):

δ = 1.37 (s, 9H, tBu); 1.50 (m, 8H, $-O-CH_2-(\underline{CH_2})_2-CH_2-O-$); 1.58 (m, 8H, 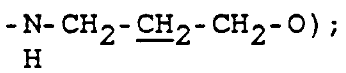); 2.15-2.37 (m, 8H, 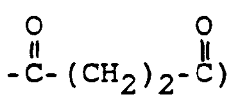); 2.79 (m, 1H, 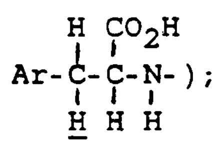);

2.88-3.12 (m, 9H, 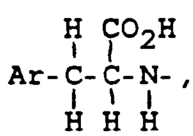, 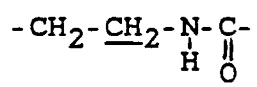);

3.32 (m, 16H, $-CH_2-\underline{CH_2}-O-$); 4.43 (m, 1H, 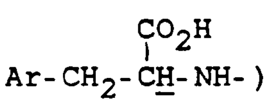); 6.55 (dd, 1H, Aryl-$H_b$); 6.70

(breit, 1H, 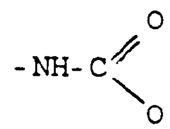); 6.82 (d, 1H, Aryl-$H_c$); 6.97 (d, 1H, Aryl-$H_a$); 7.73 (m, 3H, 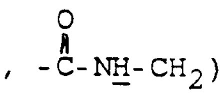); 7.78 (s, 2H, Aryl-$H_d$); 8.21 (d, 1H, 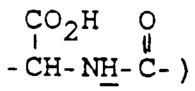); 9.95 (breit, 1H, Ar-OH); 12.70 (breit, 1H, $-CO_2H$)-

7c:

$$[C_{43}H_{65}I_3N_5O_{12}]^+ \quad [C_2F_2O_2]^-$$
$$(1224.7) \qquad (113.0)$$

MS (FAB, 3-Nitrobenzylalkohol-Matrix mit und ohne KCl-Zusatz)
m/z (%) = 1262 (18) $MK^+$; 1224 (100) $M^+$

IR (KBr): 3300 (O-H, N-H); 3080 (N-H, Amid); 3200-2400 ($-CO_2H$); 2940, 2860, 2805 (C-H); 1725

(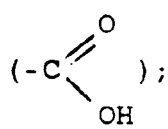);

1680, 1650

(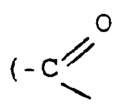, Amid I);

1560, 1540 (Amid II); 1200, 1180.

6d, 7d

aus 5b und $T_3$-Hydrochlorid analog 6a, 7a.

6d: $C_{48}H_{72}I_3N_5O_{16}$ (1355.8)

MS (FAB, 3-Nitrobenzylalkohol-Matrix mit und ohne KCl-Zusatz)
m/z (%) = 1394 (12) $MK^+$; 1378 (9) $MNa^+$ ; 1356 (22) $MH^+$ 1256 (100) $[M^+-(CH_3)_3C-O-CO + 2H]$

IR (KBr): 3380 (O-H, N-H); 3090 (N-H, Amid); 2960, 2860, 2800 (C-H); 3200-2400 ($-CO_2H$); 1740

(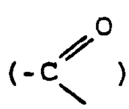);

1660

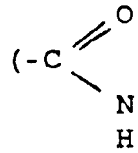 ; Amid I);

1540 (Amid II); 1370 ($C(CH_3)_3$); 1110 (-C-O-C-).

$^1$H-NMR (100-MHz, $D_4$-Methanol): δ = 1.41 (s, 9H, t-Bu); 1.50-1.92 (m, 16H, $CH_2$-$\underline{CH_2}$-$CH_2$-); 3.00-3.58 (m, 24H, $CH_2$-$\underline{CH_2}$-O-; $CH_2$-$\underline{CH_2}$-NH); 4.45

(d, 8H, 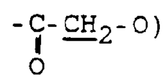);

6.54, 6.72, 6.98 (ABM-Spektrum, 3H, Aryl-$H_b$, $H_c$, -$H_a$); 7.80 ( ,2H, Aryl-$H_d$).
O-H-, N-H-Protonen durch Lösungsmittelsignale verdeckt (bzw. H/D-Austausch).

7d:

$$[C_{43}H_{65}I_3N_5O_{14}]^+ \quad [C_2F_3O_2]^-$$
$$(1256.7) \qquad (113.0)$$

MS (FAB, 3-Nitrobenzylalkohol-Matrix mit und ohne KCl-Zusatz)
m/z (%) = 1294 (11)$MK^+$; 1278 (8) $MNa^+$; 1256 (100) $MH^+$

IR (KBr): 3400 (O-H, N-H); 3070 (-$N^+H_3$, N-H); 3400-2400 (-$CO_2H$); 1740, 1720

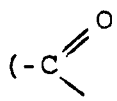); 1670 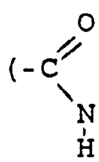, Amid I);

1560, 1540 (Amid II); 1205, 1180, 1130.

Die Polypeptid-Konjugate von 7a und 7b wurden analog Schritt 7 (Beispiel 1) hergestellt. In Schritt 7b wurden dabei 8.05 mg Verbindung 7a eingesetzt bzw. 8.20 mg Verbindung 7b. Die Markierung dieser beiden Konjugate erfolgte analog Schritt 8 (Beispiel 1).

## Patentansprüche

### Patentansprüche für folgende Vertragsstaaten : AT, BE, CH, DE, DK, FR, GB, IT, LI, NL

1. Markiertes Hapten der Formel I

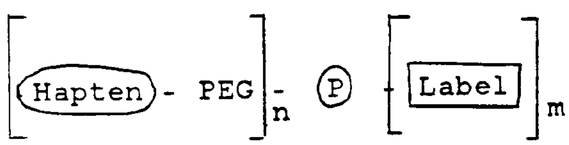 (I)

worin

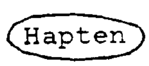

eine biologisch aktive Substanz ist,

PEG ein Polyethylenglykol der Formel II ist,

$$-\overset{O}{\overset{\|}{C}}-A-(O-CH_2-CH_2)_x-NH- \qquad (II)$$

worin

A $C_1$-$C_3$-Alkylen oder $-CH_2-NHC(O)-$ ist und

x 1 - 60 ist oder

PEG ein Rest der Formel III ist,

$$\left[-\overset{O}{\overset{\|}{C}}-A'-\overset{O}{\overset{\|}{C}}-NH-(CH_2)_o-O-(CH_2)_p-O-(CH_2)_o-NH-\right]_y \qquad (III)$$

worin

A' $C_1$-$C_3$-Alkylen oder $-CH_2-O-CH_2-$ ist und

o 2 - 4

p 1 - 5 ist und

y 1 - 7 ist,

Ⓟ ein Protein, Oligo- oder Polypeptid ist,

Label

eine mit chemischen oder physikalischen Methoden quantifizierbare Gruppe ist,

n 1 - 4 und

m 1 - 4 ist.

2. Markiertes Hapten der Formel I nach Anspruch 1, dadurch gekennzeichnet, daß

Hapten

eine Verbindung der Formel

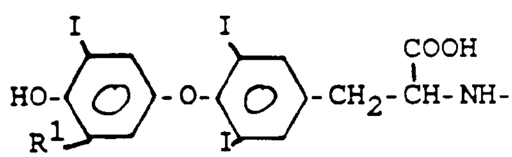

ist,

worin

$R^1$ H, I oder ein radioaktives Iodisotop ist und

PEG ein Polyethylenglykol der Formel II wie in Anspruch 1 definiert ist,

worin

A $-CH_2-$ und

x 3 - 60 ist, oder

PEG ein Rest der Formel III wie in Anspruch 1 definiert ist,

worin

o 3,

p 4 ist, y 1 oder 2 ist und

A' $-CH_2-CH_2$ oder $-CH_2-O-CH_2-$ ist,

Ⓟ ein Oligo- oder Polypeptid, HSA, BSA oder IgG oder ein IgG-Fragment ist,

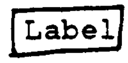


ein chemilumineszierendes Acridinium-9-(N-Sulfonyl)-carbonsäureamid-Derivat ist und

n 1 - 4 und

m 1 - 4 ist.

**3.** Verfahren zur Herstellung von Verbindungen der Formel I, dadurch gekennzeichnet, daß man das

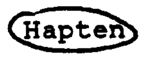


umsetzt mit einer Verbindung der Formel IV oder V

$$Z-\overset{O}{\overset{\|}{C}}-A-(O-CH_2-CH_2)_x-NH-(Sg) \qquad (IV)$$

$$Z-\left[\overset{O}{\overset{\|}{C}}-A'-\overset{O}{\overset{\|}{C}}-NH-\ (CH_2)_o-O-(CH_2)_p-O-(CH_2)_o-NH\right]_y(Sg) \qquad (V)$$

worin A, A', x, o, p und y wie in Anspruch 1 für Formel II und/oder III definiert sind und Z eine Abgangsgruppe ist und (Sg) H oder eine Aminoschutzgruppe ist und anschließend das so erhaltene Produkt

a) gegebenenfalls nach Entfernen der Aminoschutzgruppe zunächst mit einem Protein und dieses Produkt anschließend mit einem Label umsetzt oder

b) gegebenenfalls nach Entfernen der Aminoschutzgruppe mit einem Protein-Label-Konjugat umsetzt und

gegebenenfalls anschließend das Reaktionsprodukt reinigt.

**4.** Verwendung der markierten Haptene nach Anspruch 1 oder 2 in einem Immunoassay.

**5.** Verwendung nach Anspruch 4, dadurch gekennzeichnet, daß der Immunoassay ein Chemilumineszenz-Immunoassay ist.

**6.** Immunoassay zur Bestimmung von Haptenkonzentrationen in Flüssigkeiten, dadurch gekennzeichnet, daß ein mit dem zu bestimmenden Hapten spezifisch reagierender immobilisierter Antikörper mit einer Probe der zu untersuchenden Flüssigkeit, welche das zu bestimmende Hapten enthält, und einem markierten Hapten der Formel I nach Anspruch 1 inkubiert wird, die Probe und das nicht gebundene markierte Hapten von dem immobilisierten Antikörper getrennt werden und die Menge des Antikörpergebundenen markierten Haptens unter Ausnutzung der chemischen und/oder physikalischen Eigenschaften des Labels bestimmt wird und aus dieser Menge die Menge des zu bestimmenden Haptens bestimmt wird.

**7.** Immunoassay nach Anspruch 6, dadurch gekennzeichnet, daß zwischen Probenzugabe und Zugabe des markierten Haptens ein Trennschritt eingeführt wird.

**8.** Immunoassay nach Anspruch 7, dadurch gekennzeichnet, daß nach dem Trennschritt ein Waschschritt eingeführt wird.

**9.** Immunoassay nach Anspruch 6, 7 oder 8, dadurch gekennzeichnet, daß der Immunoassay ein Chemilumineszenz-Immunoassay ist und das Label ein chemilumineszierendes Label ist.

**10.** Immunoassy nach Anspruch 9, dadurch gekennzeichnet, daß ein markiertes Hapten der Formel I nach

Anspruch 2 eingesetzt wird.

**11.** Testkit zur Durchführung eines Immunoassays, dadurch gekennzeichnet, daß er einen auf einer Festphase immobilisierten Antikörper und ein markiertes Hapten der Formel I nach Anspruch 1 enthält.

**Patentansprüche für folgenden Vertragsstaat: ES**

**1.** Verfahren zur Herstellung eines markierten Haptens der Formel I

$$\left[\text{Hapten - PEG}\right]_n - \text{P} - \left[\text{Label}\right]_m \qquad (I)$$

worin

| | |
|---|---|
| Hapten | eine biologisch aktive Substanz ist, |
| PEG | ein Polyethylenglykol der Formel II ist, |

$$-\overset{O}{\overset{\|}{C}}-A-(O-CH_2-CH_2)_x-NH- \qquad (II)$$

worin

| | |
|---|---|
| A | $C_1$-$C_3$-Alkylen oder -$CH_2$-NHC(O)- ist und |
| x | 1 - 60 ist, oder |

PEG ein Rest der Formel III ist,

$$\left[-\overset{O}{\overset{\|}{C}}-A'-\overset{O}{\overset{\|}{C}}-NH-\ (CH_2)_o-O-(CH_2)_p-O-(CH_2)_o-NH-\right]_y \qquad (III)$$

worin

| | |
|---|---|
| A' | $C_1$-$C_3$-Alkylen oder -$CH_2$-O-$CH_2$- ist und |
| o | 2 - 4 |
| p | 1 - 5 ist und |
| y | 1 - 7 ist, |

| | |
|---|---|
| P | ein Protein, Oligo- oder Polypeptid ist, |
| Label | eine mit chemischen oder physikalischen Methoden quantifizierbare Gruppe ist, |
| n | 1 - 4 und |
| m | 1 - 4 ist, |

in dem man das Hapten umsetzt mit
einer Verbindung der Formel IV oder V

$$Z-\overset{O}{\overset{\|}{C}}-A-(O-CH_2-CH_2)_x-NH-Sg \qquad (IV)$$

$$Z\left[-\overset{O}{\overset{\|}{C}}-A'-\overset{O}{\overset{\|}{C}}-NH-\ (CH_2)_o-O-(CH_2)_p-O-(CH_2)_o-NH-\right]_y(Sg) \qquad (V)$$

worin A, A', x, o, p und y wie in Anspruch 1 für Formel II und/oder III definiert sind und Z eine Abgangsgruppe ist und (Sg) H oder eine Aminoschutzgruppe ist und anschließend das so erhaltene Produkt

a) gegebenenfalls nach Entfernen der Aminoschutzgruppe zunächst mit einem Protein und dieses Produkt anschließend mit einem Label umsetzt oder
b) gegebenenfalls nach Entfernen der Aminoschutzgruppe mit einem Protein-Label-Konjugat umsetzt und

gegebenenfalls anschließend das Reaktionsprodukt reinigt.

2. Verfahren zur Herstellung eines markierten Haptens nach Anspruch 1, dadurch gekennzeichnet, daß das Hapten eine Verbindung der Formel

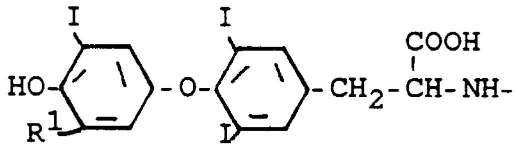

ist,

worin
$R^1$ H, I oder ein radioaktives Iodisotop ist und

PEG ein Polyethylenglykol der Formel II wie in Anspruch 1 definiert ist,
worin
A $-CH_2-$ und
x 3 - 60 ist, oder

PEG ein Rest der Formel III wie in Anspruch 1 definiert ist,
worin
o 3,
p 4 ist, y 1 oder 2 ist und
A' $-CH_2-CH_2$ oder $-CH_2-O-CH_2-$ ist,

P ein oligo- oder Polypeptid, HSA, BSA oder IgG oder ein IgG-Fragment ist,
Label ein chemilumineszierendes Acridinium-9-(N-Sulfonyl)-carbonsäureamid-Derivat ist und
n 1 - 4 und
m 1 - 4 ist.

3. Verwendung der markierten Haptene nach Anspruch 1 oder 2 in einem Immunoassay.

4. Verwendung nach Anspruch 3, dadurch gekennzeichnet, daß der Immunoassay ein Chemilumineszenz-Immunoassay ist.

5. Immunoassay zur Bestimmung von Haptenkonzentrationen in Flüssigkeiten, dadurch gekennzeichnet, daß ein mit dem zu bestimmenden Hapten spezifisch reagierender immobilisierter Antikörper mit einer Probe der zu untersuchenden Flüssigkeit, welche das zu bestimmende Hapten enthält, und einem markierten Hapten der Formel I nach Anspruch 1 inkubiert wird, die Probe und das nicht gebundene markierte Hapten von dem immobilisierten Antikörper getrennt werden und die Menge des Antikörpergebundenen markierten Haptens unter Ausnutzung der chemischen und/oder physikalischen Eigenschaften des Labels bestimmt wird und aus dieser Menge die Menge des zu bestimmenden Haptens bestimmt wird.

6. Immunoassay nach Anspruch 5, dadurch gekennzeichnet, daß zwischen Probenzugabe und Zugabe des markierten Haptens ein Trennschritt eingeführt wird.

7. Immunoassay nach Anspruch 6, dadurch gekennzeichnet, daß nach dem Trennschritt ein Waschschritt eingeführt wird.

8. Immunoassay nach Anspruch 5, 6 oder 7, dadurch gekennzeichnet, daß der Immunoassay ein Chemilumineszenz-Immunoassay ist und das Label ein chemilumineszierend Label ist.

9. Immunoassay nach Anspruch 8, dadurch gekennzeichnet, daß ein markiertes Hapten der Formel I nach Anspruch 2 eingesetzt wird.

10. Testkit zur Durchführung eines Immunoassays, dadurch gekennzeichnet, daß er einen auf einer Festphase immobilisierten Antikörper und ein markiertes Hapten der Formel I nach Anspruch 1 enthält.

## Claims

### Claims for the following Contracting States : AT, BE, CH, DE, DK, FR, GB, IT, LI, NL

1. A labeled hapten of the formula I

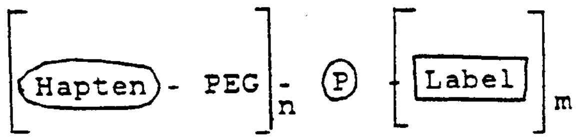 (I)

in which

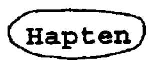

is a biologically active substance,

PEG is a polyethylene glycol of the formula II

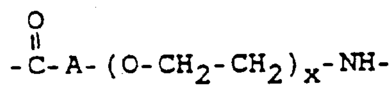 (II)

in which
A is $C_1$-$C_3$-alkylene or $-CH_2-NHC(O)-$ and
x is 1 - 60, or

PEG is a radical of the formula III

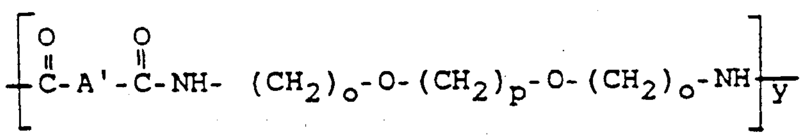 (III)

in which
A′ is $C_1$-$C_3$-alkylene or $-CH_2-O-CH_2-$ and
o is 2 - 4
p is 1 - 5 and
y is 1 - 7,

Ⓟ is a protein, oligopeptide or polypeptide,

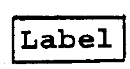

is a group quantifiable by chemical or physical methods,
n is 1 - 4 and
m is 1 - 4.

2. A labeled hapten of the formula I as claimed in claim 1, wherein

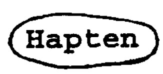

is a compound of the formula

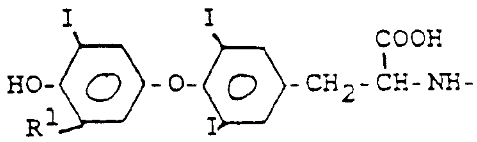

in which

$R^1$ is H, I or a radioactive iodine isotope and

PEG is a polyethylene glycol of the formula II as is defined in claim 1,
in which
A is $-CH_2-$ and
x is 3 - 60, or

PEG is a radical of the formula III as is defined in claim 1,
in which
o is 3,
p is 4, y is 1 or 2 and
A′ is $-CH_2-CH_2-$ or $-CH_2-O-CH_2-$,

Ⓟ is an oligopeptide or polypeptide, HSA, BSA or IgG or an IgG fragment,

Label

is a chemiluminescent acridinium-9-(N-sulfonyl)-carboxamide derivative and

n is 1 - 4 and

m is 1- 4.

3. A process for the preparation of compounds of the formula I, which comprises reacting the

Hapten

with a compound of the formula IV or V

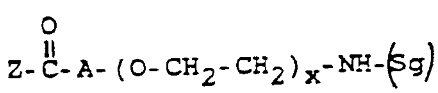 (IV)

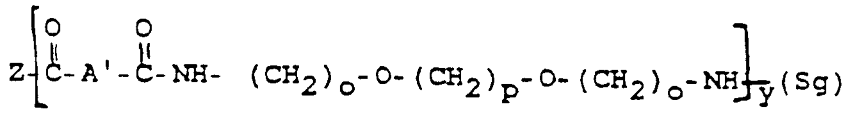 (V)

in which A, A′, x, o, p and y are as defined in claim 1 for formula II and/or III and Z is a leaving group and (Sg) is H or an amino protective group, and then reacting the product thus obtained

a) first with a protein, if appropriate after removing the amino protective group, and then reacting this product with a label or

b) reacting with a protein-label conjugate, if appropriate after removing the amino protective group, and if appropriate then purifying the reaction product.

4. The use of the labeled haptens as claimed in claim 1 or 2 in an immunoassay.

5. The use as claimed in claim 4, wherein the immunoassay is a chemiluminescence immunoassay.

6. An immunoassay for the determination of hapten concentrations in liquids, which comprises incubating an immobilized antibody specifically reacting with the hapten to be determined with a sample of the liquid

to be investigated, which contains the hapten to be determined, and a labeled hapten of the formula I as claimed in claim 1, separating the sample and the unbound labeled hapten from the immobilized antibody and determining the amount of the antibody-bound labeled hapten utilizing the chemical and/or physical properties of the label and from this amount determining the amount of the hapten to be determined.

7. An immunoassay as claimed in claim 6, wherein a separation step is inserted between sample addition and addition of the labeled hapten.

8. An immunoassay as claimed in claim 7, wherein a washing step is inserted after the separation step.

9. An immunoassay as claimed in claim 6, 7 or 8, wherein the immunoassay is a chemiluminescent immunoassay and the label is a chemiluminescent label.

10. An immunoassay as claimed in claim 9, wherein a labeled hapten of the formula I as claimed in claim 2 is employed.

11. A test kit for carrying out an immunoassay, which contains an antibody immobilized on a solid phase and a labeled hapten of the formula I as claimed in claim 1.

**Claims for the following Contracting State : ES**

1. A process for the preparation of a labeled hapten of the formula I

$$\left[\text{Hapten - PEG}\right]_n \text{- P -} \left[\text{Label}\right]_m \qquad (I)$$

in which
Hapten is a biologically active substance,
PEG is a polyethylene glycol of the formula II

$$-\overset{O}{\overset{\|}{C}}-A-(O-CH_2-CH_2)_x-NH- \qquad (II)$$

in which
A is $C_1$-$C_3$-alkylene or $-CH_2-NHC(O)-$ and
x is 1 - 60, or
PEG is a radical of the formula III

$$\left[-\overset{O}{\overset{\|}{C}}-A'-\overset{O}{\overset{\|}{C}}-NH-(CH_2)_o-O-(CH_2)_p-O-(CH_2)_o-NH-\right]_y \qquad (III)$$

in which
A' is $C_1$-$C_3$-alkylene or $-CH_2-O-CH_2-$ and
o is 2 - 4
p is 1 - 5 and
y is 1 - 7,
P is a protein, oligopeptide or polypeptide,
Label is a group quantifiable by chemical or physical methods,
n is 1 - 4 and
m is 1 - 4,
in which the hapten is reacted with
a compound of the formula IV or V

$$Z-\overset{O}{\overset{\|}{C}}-A-(O-CH_2-CH_2)_x-NH-Sg \qquad (IV)$$

$$Z\left[\overset{O}{\overset{\|}{C}}-A'-\overset{O}{\overset{\|}{C}}-NH-\ (CH_2)_o-O-(CH_2)_p-O-(CH_2)_o-NH\right]_y(Sg) \qquad (V)$$

in which A, A′, x, o, p and y are as defined in claim 1 for formula II and/or III and Z is a leaving group and (Sg) is H or an amino protective group, and then reacting the product thus obtained

a) first with a protein, if appropriate after removing the amino protective group, and then reacting this product with a label or

b) reacting with a protein-label conjugate, if appropriate after removing the amino protective group, and if appropriate then purifying the reaction product.

2. A process for the preparation of a labeled hapten as claimed in claim 1, wherein the hapten is a compound of the formula

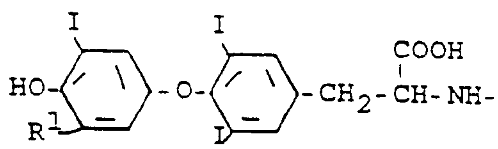

in which

$R^1$ is H, I or a radioactive iodine isotope and

| | | |
|---|---|---|
| PEG | is a polyethylene glycol of the formula II as is defined in claim 1, in which | |
| | A | is $-CH_2-$ and |
| | x | is 3 - 60, or |
| PEG | is a radical of the formula III as is defined in claim 1, in which | |
| | o | is 3, |
| | p | is 4, y is 1 or 2 and |
| | A′ | is $-CH_2-CH_2-$ or $-CH_2-O-CH_2-$, |
| P | is an oligopeptide or polypeptide, HSA, BSA or IgG or an IgG fragment, | |
| Label | is a chemiluminescent acridinium-9-(N-sulfonyl)-carboxamide derivative and | |
| n | is 1 - 4 and | |
| m | is 1 - 4. | |

3. The use of the labeled haptens as claimed in claim 1 or 2 in an immunoassay.

4. The use as claimed in claim 3, wherein the immunoassay is a chemiluminescence immunoassay.

5. An immunoassay for the determination of hapten concentrations in liquids, which comprises incubating an immobilized antibody specifically reacting with the hapten to be determined with a sample of the liquid to be investigated, which contains the hapten to be determined, and a labeled hapten of the formula I as claimed in claim 1, separating the sample and the unbound labeled hapten from the immobilized antibody and determining the amount of the antibody-bound labeled hapten utilizing the chemical and/or physical properties of the label and from this amount determining the amount of the hapten to be determined.

6. An immunoassay as claimed in claim 5, wherein a separation step is inserted between sample addition and addition of the labeled hapten.

7. An immunoassay as claimed in claim 6, wherein a washing step is inserted after the separation step.

8. An immunoassay as claimed in claim 5, 6 or 7, wherein the immunoassay is a chemiluminescence immunoassay and the label is a chemiluminesceny label.

**9.** An immunoassay as claimed in claim 8, wherein a labeled hapten of the formula I as claimed in claim 2 is employed.

**10.** A test kit for carrying out an immunoassay, which contains an antibody immobilized on a solid phase and a labeled hapten of the formula I as claimed in claim 1.

## Revendications

**Revendications pour les Etats contractants suivants : AT, BE, CH, DE, DK, FR, GB, IT, LI, NL**

**1.** Haptène marqué de formule I

$$\left[\text{haptène} - \text{PEG} - \right]_n - (\text{P}) - \left[\boxed{\text{marqueur}}\right]_m \qquad (\text{I})$$

dans laquelle

haptène

est une substance biologiquement active,

PEG est un polyéthylèneglycol de formule II

$$-\overset{\overset{\displaystyle O}{\|}}{C}-A-(O-CH_2-CH_2)_x-NH- \qquad (\text{II})$$

dans laquelle

A est un groupe alkylène en $C_1$-$C_3$ ou $-CH_2-NHC(O)-$ et

x va de 1 à 60, ou

PEG est un radical de formule III

$$-\left[-\overset{\overset{\displaystyle O}{\|}}{C}-A'-\overset{\overset{\displaystyle O}{\|}}{C}-NH-(CH_2)_o-O-(CH_2)_p-O-(CH_2)_o-NH\right]_y- \qquad (\text{III})$$

dans laquelle

A' est un groupe alkylène en $C_1$-$C_3$ ou $-CH_2-O-CH_2-$ et

o va de 2 à 4,

p va de 1 à 5 et

y va de 1 à 7;

Ⓟ est une protéine, un oligopeptide ou un poly-peptide,

marqueur

est un groupe quantitativement déterminable par des méthodes chimiques ou physiques;

n va de 1 à 4 et

m va de 1 à 4.

**2.** Haptène marqué de formule I selon la revendication 1, caractérisé en ce que

haptène

est un radical de formule

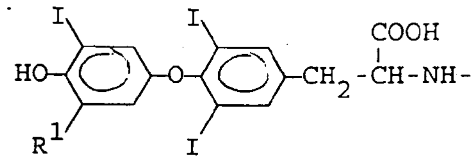

dans laquelle
$R^1$ est H, I ou un isotope d'iode radioactif, et

PEG est un polyéthylèneglycol de formule II, telle que définie dans la revendication 1, dans lequel
- A est $-CH_2-$ et
- x va de 3 à 60, ou

PEG est un radical de formule III, telle que définie dans la revendication 1, dans lequel
- o est 3 et
- p est 4,
- y est 1 ou 2 et
- A' est $CH_2-CH_2$ ou $CH_2-O-CH_2$,

Ⓟ est un oligopeptide ou polypeptide, ASH (albumine de sérum humain), ASB (albumine de sérum bovin), une IgG ou un fragment d'IgG,

marqueur

est un dérivé chimiluminescent d'acridinium-9-(N-sulfonyl)carboxamide, et

n va de 1 à 4 et

m va de 1 à 4.

3. Procédé pour la préparation de composés de formule I, caractérisé en ce que l'on fait réagir l'haptène avec un composé de formule IV ou V

$$Z-\overset{O}{\overset{\|}{C}}-A-(-O-CH_2-CH_2-)_x-NH(Sg) \qquad (IV)$$

$$Z-\left[-\overset{O}{\overset{\|}{C}}-A'-\overset{O}{\overset{\|}{C}}-NH-(CH_2)_o-O-(CH_2)_p-O(CH_2)_o-NH-\right]_y-(Sg) \qquad (V)$$

formules dans lesquelles A, A', x, o, p et y sont tels que définis dans la revendication 1 pour la formule II et/ou la formule III, et Z est un groupe partant et (Sg) est H ou un groupe protecteur de fonction amino, et ensuite on fait réagir le produit :

a) éventuellement après élimination du groupe protecteur de fonction amino, d'abord avec une protéine, et on fait ensuite réagir ce produit avec un marqueur, ou

b) éventuellement après élimination du groupe protecteur de fonction amino, avec un conjugué protéine-marqueur,

et éventuellement on purifie ensuite le produit de réaction.

4. Utilisation des haptènes marqués selon la revendication 1 ou 2, dans un essai immunologique.

5. Utilisation selon la revendication 4, caractérisé en ce que l'essai immunologique est un essai immunologique par chimiluminescence.

6. Essai immunologique pour la détermination de concentrations d'haptènes dans des liquides, caractérisé en ce que l'on met à incuber un anticorps immobilisé, réagissant spécifiquement avec l'haptène à déterminer, avec un échantillon du liquide à étudier, qui contient l'haptène à déterminer, et avec un haptène marqué de formule I selon la revendication 1, on sépare l'échantillon et l'haptène marqué non fixé d'avec l'anticorps immobilisé, et on détermine la quantité de l'haptène marqué, lié à l'anticorps, en utilisant les propriétés physiques et/ou chimiques du marqueur, et on détermine à partir de cette quantité la quantité de l'haptène à déterminer.

**7.** Essai immunologique selon la revendication 6, caractérisé en ce qu'une étape de séparation est introduite entre l'addition de l'échantillon et l'addition de l'haptène marqué.

**8.** Essai immunologique selon la revendication 7, caractérisé en ce qu'une étape de lavage est introduite après l'étape de séparation.

**9.** Essai immunologique selon la revendication 6, 7 ou 8, caractérisé en ce que l'essai immunologique est un essai immunologique par chimiluminescence et le marqueur est un marqueur chimiluminescent.

**10.** Essai immunologique selon la revendication 9, caractérisé en ce que l'on utilise un haptène marqué de formule I selon la revendication 2.

**11.** Nécessaire d'essai pour l'exécution d'un essai immunologique, caractérisé en ce qu'il contient un anticorps immobilisé sur une phase solide et un haptène marqué de formule I selon la revendication 1.

**Revendications pour l'Etat contractant suivant : ES**

**1.** Procédé pour la préparation d'un haptène marqué de formule I

$$\left[\text{haptène} - \text{PEG} -\right]_n - (P) - \left[\boxed{\text{marqueur}}\right]_m \qquad (I)$$

dans laquelle

(haptène) est une substance biologiquement active,

PEG est un polyéthylèneglycol de formule II

$$-\overset{O}{\overset{\|}{C}}-A-(O-CH_2-CH_2)_x-NH- \qquad (II)$$

dans laquelle
A est un groupe alkylène en $C_1$-$C_3$ ou $-CH_2-NHC(O)-$ et
x va de 1 à 60, ou

PEG est un radical de formule III

$$-\left[-\overset{O}{\overset{\|}{C}}-A'-\overset{O}{\overset{\|}{C}}-NH-(CH_2)_o-O-(CH_2)_p-O-(CH_2)_o-NH\right]_y- \qquad (III)$$

dans laquelle
A' est un groupe alkylène en $C_1$-$C_3$ ou $-CH_2-O-CH_2-$ et
o va de 2 à 4 et p va de 1 à 5, et
y va de 1 à 7;

Ⓟ est une protéine, un oligopeptide ou un polypeptide,

$\boxed{\text{marqueur}}$ est un groupe quantitativement déterminable par des méthodes chimiques ou physiques;

n va de 1 à 4 et

m va de 1 à 4,

dans lequel on fait réagir l'haptène avec un composé de formule IV ou V

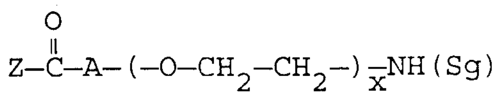

(IV)

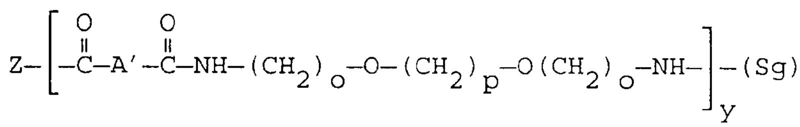

(V)

formules dans lesquelles A, A', x, o, p et y sont tels que définis plus haut pour la formule II et/ou la formule III, et Z est un groupe partant et (Sg) est H ou un groupe protecteur de fonction amino, et ensuite on fait réagir le produit obtenu

a) éventuellement après élimination du groupe protecteur de fonction amino, d'abord avec une protéine, et on fait ensuite réagir ce produit avec un marqueur, ou

b) éventuellement après élimination du groupe protecteur de fonction amino, avec un conjugué protéine-marqueur,

et éventuellement on purifie ensuite le produit de réaction.

2. Procédé pour la préparation d'un haptène marqué selon la revendication 1, caractérisé en ce que

(haptène)

est un radical de formule

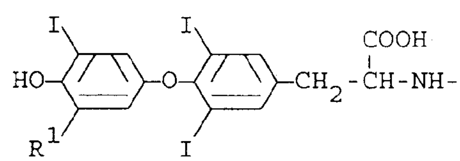

dans laquelle

$R^1$ est H, I ou un isotope d'iode radioactif, et

PEG est un polyéthylèneglycol de formule II, telle que définie dans la revendication 1, dans lequel

A est $-CH_2-$ et

x va de 3 à 60, ou

PEG est un radical de formule III, telle que définie dans la revendication 1, dans lequel

o est 3 et

p est 4,

y est 1 ou 2 et

A' est $CH_2-CH_2$ ou $CH_2-O-CH_2$,

Ⓟ est une protéine, un oligopeptide ou polypeptide, ASH (albumine de sérum humain), ASB (albumine de sérum bovin), une IgG ou un fragment d'IgG,

[marqueur]

est un dérivé chimiluminescent d'acridinium-9-(N-sulfonyl)carboxamide, et

n va de 1 à 4 et

m va de 1 à 4.

3. Utilisation des haptènes marqués selon la revendication 1 ou 2, dans un essai immunologique.

4. Utilisation selon la revendication 3, caractérisé en ce que l'essai immunologique est un essai immunologique par chimiluminescence.

5. Essai immunologique pour la détermination de concentrations d'haptènes dans des liquides, caractérisé en ce que l'on met à incuber un anticorps immobilisé, réagissant spécifiquement avec l'haptène à déterminer, avec un échantillon du liquide à étudier, qui contient l'haptène à déterminer, et avec un haptène marqué de formule I selon la revendication 1, on sépare l'échantillon et l'haptène marqué non fixé d'avec l'anticorps immobilisé, et on détermine la quantité de l'haptène marqué, lié à l'anticorps, en utilisant les propriétés physiques et/ou chimiques du marqueur, et on détermine à partir de cette quantité la quantité de l'haptène à déterminer.

6. Essai immunologique selon la revendication 5, caractérisé en ce qu'une étape de séparation est introduite entre l'addition de l'échantillon et l'addition de l'haptène marqué.

7. Essai immunologique selon la revendication 6, caractérisé en ce qu'une étape de lavage est introduite après l'étape de séparation.

8. Essai immunologique selon la revendication 5, 6 ou 7, caractérisé en ce que l'essai immunologique est un essai immunologique par chimiluminescence et le marqueur est un marqueur chimiluminescent.

9. Essai immunologique selon la revendication 8, caractérisé en ce que l'on utilise un haptène marqué de formule I selon la revendication 2.

10. Nécessaire d'essai pour l'exécution d'un essai immunologique, caractérisé en ce qu'il contient un anticorps immobilisé sur une phase solide et un haptène marqué de formule I selon la revendication 1.

# Syntheseschema 1

$HO(CH_2\text{-}CH_2O)_xH = HO(CH_2\text{-}CH_2\text{-}O)_{x-1}CH_2\text{-}CH_2\text{-}OH$

$= (HO)_2(CH_2\text{-}CH_2\text{-}O)_{x-1}(CH_2\text{-}CH_2)$

$(Cl)_s(CH_2\text{-}CH_2O)_{x-1}(CH_2\text{-}CH_2)(OH)_z$

$s,z = 0,1,2 \quad s+z= 2$

(VII) AG-A-COR$^2$

$(Cl)_s(CH_2\text{-}CH_2O)_{x-1}(CH_2\text{-}CH_2)(OA\text{-}COR^2)_z$

$= (Cl)_s(CH_2\text{-}CH_2O)_x(A\text{-}C(O)R^2)_z$ VI

$(N_3)_s(CH_2\text{-}CH_2O)_x(A\text{-}CO_2H)_z$ VIII

$N_3(CH_2\text{-}CH_2O)_xA\text{-}CO_2H$ $s=z=1$

$H_2N(CH_2\text{-}CH_2O)_xACO_2H$

$(Sg)HN(CH_2\text{-}CH_2O)_xA\text{-}COZ$

Z= OH, -Cl , 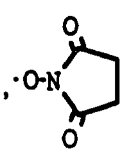 , 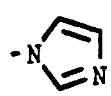 , 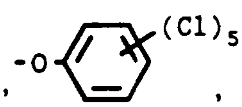 ,

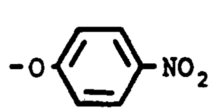 , 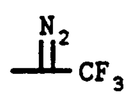 , 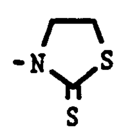

Sg= H oder Aminoschutzgruppe

## Syntheseschema 2

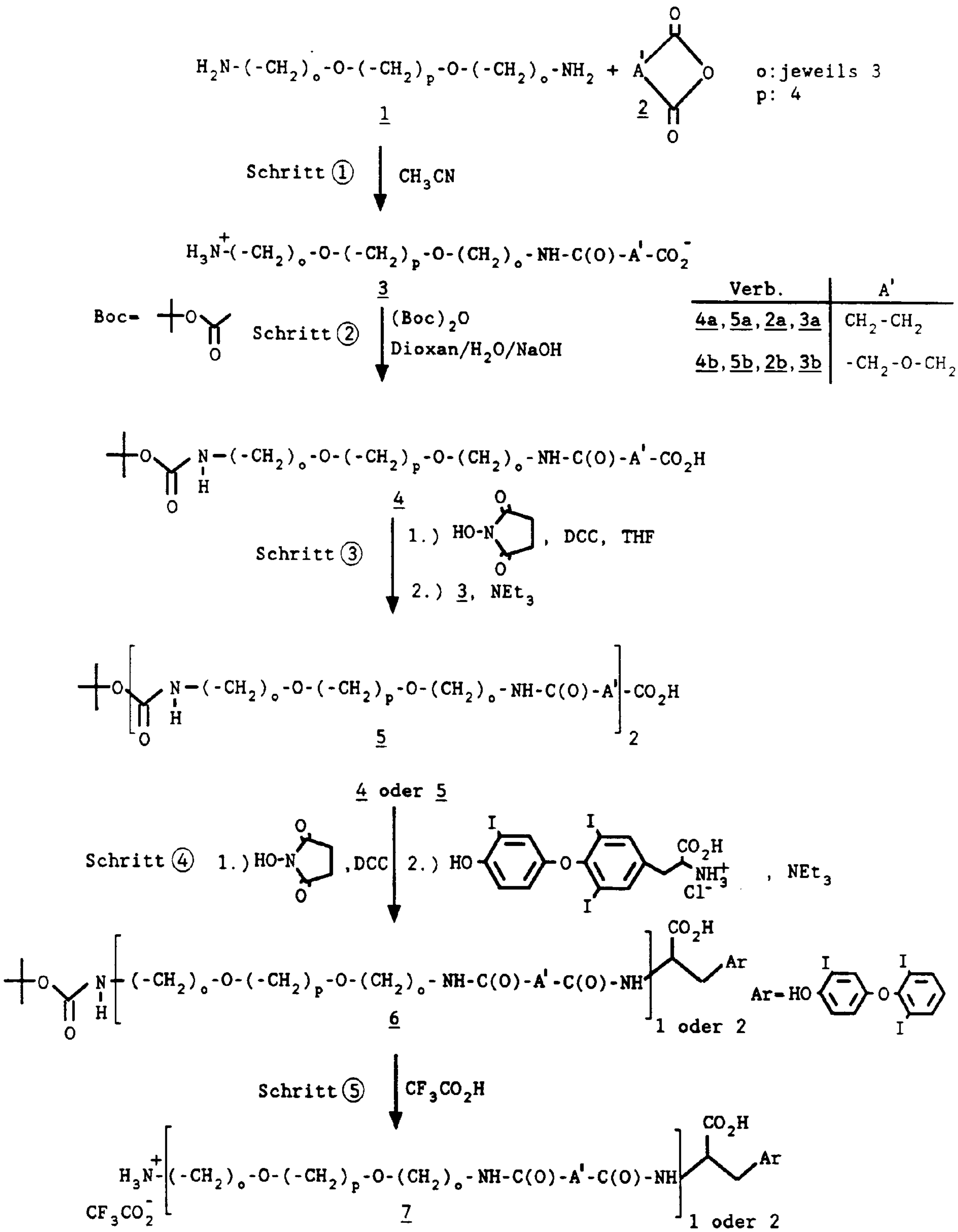


| Verb. | A' |
|---|---|
| 4a, 5a, 2a, 3a | $CH_2$-$CH_2$ |
| 4b, 5b, 2b, 3b | -$CH_2$-O-$CH_2$ |

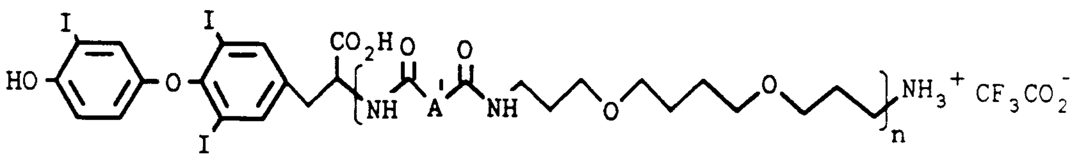

| Verb. | A' | n |
|---|---|---|
| 6a 7a | $(CH_2)_2$ | 1 |
| 6b 7b | $CH_2$-O-$CH_2$ | 1 |
| 6c 7c | $(CH_2)_2$ | 2 |
| 6d 7d | $CH_2$-O-$CH_2$ | 2 |